# EUROPEAN PATENT APPLICATION

(11) **EP 4 357 354 A1**
(43) Date of publication of application: **24.04.2024**
(21) Application number: 22825079.1
(22) Date of filing: 17.06.2022
(51) Int. Cl.: C07K 7/06, A61K 39/00, A61P 25/00, A61P 25/16, A61P 25/28, C07K 19/00, C07K 14/47, C12N 15/12

(54) **EPITOPE PEPTIDE OF HUMAN ALPHA-SYNUCLEIN AND PHARMACEUTICAL COMPOSITION INCLUDING SAID PEPTIDE**

(30) Priority: 18.06.2021 JP 2021101745
(71) Applicant: Sumitomo Pharma Co., Ltd., Osaka 541-0045 (JP)
(72) Inventor: HASEZAKI, Takuya, Suita-shi, Osaka 564-0053 (JP); YAMADA, Kazuto, Osaka-shi, Osaka 541-0045 (JP); MATSUMOTO, Mitsuhiro, Osaka-shi, Osaka 554-0022 (JP)
(74) Representative: Hoffmann Eitle
(86) International application number: PCT/JP2022/024263
(87) International publication number: WO 2022/265091

(57) **Abstract**

An object of the present invention is to provide an epitope peptide of human α-synuclein and a pharmaceutical composition comprising the peptide which have a high antibody inducibility with respect to not only α-synuclein monomers but also α-synuclein oligomers. A polypeptide of the present invention consists of any of the following amino acid sequences: (a) an amino acid sequence set forth in SEQ ID NO: 1; (b) an amino acid sequence set forth in SEQ ID NO: 2; and (c) an amino acid sequence in which one, two, or three amino acids are substituted, deleted, added, and/or inserted in the amino acid sequence set forth in SEQ ID NO: 1 or SEQ ID NO: 2.

## Description

### Technical Field

The present invention relates to an epitope peptide of human α-synuclein, and a pharmaceutical composition comprising the peptide.

### Background Art

Human α-synuclein (αSyn) is a protein that is encoded by a SNCA gene, consists of 140 amino acids, has a molecular weight of 14.5 kDa, and is highly expressed in the central nervous system, particularly in the cerebral cortex, olfactory bulb, hippocampus, dentate gyrus, corpus striatum, thalamus, and cerebellum. α-Synuclein is thought to be involved in synaptic activities such as the transport of synaptic vesicles and the control of neurotransmitter release, and in the control of dopamine neurotransmission, but the full picture of its functions has not yet been elucidated. Meanwhile, α-synuclein is also known as a major constituent protein of Lewy bodies which are pathological structures characteristic of the brains of patients with various neurodegenerative diseases. Lewy bodies are abnormal circular structures (inclusion bodies) found inside nerve cells.

Synucleinopathies (also called α-synucleinopathies) are neurodegenerative diseases characterized by the abnormal accumulation of aggregates of α-synuclein protein in nerve cells, nerve fibers, or glial cells, and examples thereof primarily comprise Parkinson's disease (PD), dementia with Lewy bodies (DLB), and multiple system atrophy (MSA) (Non-Patent Literature 1).

Although the exact cause of PD is still unknown, in recent years, Braak's hypothesis stating that pathological α-synuclein propagates from the olfactory bulb and the vagus nerve as origin points to the cerebral cortex as the disease progresses has been accepted (Non-Patent Literature 2).

Focusing on the accumulation of α-synuclein, attempts at immunotherapy of synucleinopathies such as PD using antibodies to α-synuclein have been reported. For example, Patent Literature 1 discloses a pharmaceutical composition for preventing or treating a Lewy body disease, the pharmaceutical composition comprising α-synuclein, an immunogenic fragment of α-synuclein, an antibody to α-synuclein, or an antibody to an immunogenic fragment of α-synuclein, in which the immunogenic fragment comprises amino acids 130 to 140 of α-synuclein and has less than 40 amino acids. Furthermore, epitope peptides or mimotope peptides of α-synuclein which induce antibodies to α-synuclein have also been reported (Patent Literature 2 and Patent Literature 3).

Meanwhile, it is known that a vaccine having a higher antibody-inducing ability is provided by linking a carrier protein to an antigen to form a conjugate vaccine. Carrier proteins that can be used in the conjugate vaccine preferably have immunostimulating properties and low antigenicity, and examples thereof comprise an OSK-1 peptide and KLH disclosed in Patent Literature 4.

### Citation List

### Patent Literature

[Patent Literature 1] PCT International Publication No. WO2004-041067
[Patent Literature 2] PCT International Publication No. WO2009-103105
[Patent Literature 3] PCT International Publication No. WO2018-232369
[Patent Literature 4] PCT International Publication No. WO2017-164409

[Non-Patent Literature 1] Maria J Marti et al., "Clinical overview of the synucleinopathies," Mov Disord., Suppl 6: S21-7, 2003
[Non-Patent Literature 2] Glenda Halliday et al., "Milestones in Parkinson's Disease-Clinical and Pathologic Features," Mov. Disord. 26, 1051-1021 (2011)
[Non-Patent Literature 3] Hodaka Yamakado et al., "α-Synuclein BAC transgenic mice as a model for Parkinson's disease manifested decreased anxiety-like behavior and hyperlocomotion," Neuro science Research, Volume 73, Issue 2, 173-177 (2012)
[Non-Patent Literature 4] Katsuya Araki et al., "A small-angle X-ray scattering study of alpha-synuclein from human red blood cells," Sci Rep.2016, 6:30473. doi: 10.1038/srep30473.

### Summary of Invention

### Technical Problem

Although antibodies to monomers of α-synuclein are induced depending on conventional epitopes or mimotopes, the induction of antibodies to α-synuclein aggregates (α-synuclein oligomers), which are the cause of synucleinopathies, is unknown. In addition, because in the periphery, α-synuclein is expressed as a monomer in B cells, T cells, NK cells, monocytes, and blood cells comprising platelets and red blood cells (Non-Patent Literature 4), an antibody that binds to a monomer is thought to be captured in the periphery and does not migrate to the brain when an antibody to α-synuclein is tried as a therapeutic agent. Therefore, when an antibody to α-synuclein is tried as a therapeutic agent, it is thought that using an antibody having a binding ability to oligomers is more desirable.

An object of the present invention is to provide an epitope peptide of human α-synuclein and a pharmaceutical composition comprising the peptide which have a high ability to induce antibodies not only to α-synuclein monomers but also to α-synuclein oligomers.

### Solution to Problem

The inventors of the present invention found that, when a polypeptide consisting of an amino acid sequence set forth in SEQ ID NO: 1 or SEQ ID NO: 2 and a mimic thereof (mimotope) is administered to mice, an anti-α-synuclein monomer antibody and an anti-synuclein oligomer antibody are induced, which led to the completion of the present invention.

Specifically, the present invention relates to each of the following inventions.
[1] A polypeptide consisting of any of the following amino acid sequences:
   (a) an amino acid sequence set forth in SEQ ID NO: 1;
   (b) an amino acid sequence set forth in SEQ ID NO: 2; and
   (c) an amino acid sequence in which one, two, or three amino acids are substituted, deleted, added, and/or inserted in the amino acid sequence set forth in SEQ ID NO: 1 or SEQ ID NO: 2.
[2] The polypeptide according to [1], in which the amino acid sequence is the amino acid sequence set forth in SEQ ID NO: 1.
[3] The polypeptide according to [1], in which the amino acid sequence is the amino acid sequence set forth in SEQ ID NO: 2.
[4] The polypeptide according to [1], in which the amino acid sequence is an amino acid sequence set forth in any one of SEQ ID NOs: 4 to 9 and SEQ ID NOs: 22 to 24.
[5] The polypeptide according to [1], in which the amino acid sequence is an amino acid sequence set forth in any one of SEQ ID NO: 3, SEQ ID NO: 10, SEQ ID NO: 11, and SEQ ID NOs: 18 to 21.
[6] A conjugate comprising the polypeptide according to any one of [1] to [5] and a carrier protein.
[7] The conjugate according to [6], in which the carrier protein is keyhole limpet hemocyanin (KLH).
[8] The conjugate according to [6], in which the carrier protein is a peptide (AJ peptide) consisting of an amino acid sequence identical or substantially identical to an amino acid sequence set forth in SEQ ID NO: 13.
[9] The conjugate according to [8], in which the AJ peptide and the polypeptide are linked via ε-aminocaproic acid and/or a peptide linker.
[10] The conjugate according to [9], in which the peptide linker is a peptide linker consisting of one to six L-amino acids or a repeating sequence thereof.
[11] The conjugate according to any one of [8] to [10], in which an N-terminal amino acid of the conjugate is acetylated.
[12] The conjugate according to any one of [8] to [11], in which a C-terminal amino acid of the conjugate is amidated.
[13] A pharmaceutical composition comprising the polypeptide according to any one of [1] to [5] or the conjugate according to any one of [6] to [12].
[14] The pharmaceutical composition according to [13], further comprising an adjuvant.
[15] The pharmaceutical composition according to [13] or [14], which is a vaccine for preventing, ameliorating, or treating a neurodegenerative disease.
[16] The pharmaceutical composition according to [15], in which the neurodegenerative disease is a disease associated with α-synuclein accumulation.
[17] The pharmaceutical composition according to [16], in which the disease associated with the α-synuclein accumulation is Parkinson's disease.
[18] A nucleic acid which encodes the polypeptide according to any one of [1] to [5] or the conjugate according to [8] or [10].
[19] An expression vector comprising the nucleic acid according to [18].
[20] A recombinant cell which is obtained by transforming a host cell with the expression vector according to [19].
[21] An antibody which specifically binds to the polypeptide according to any one of [1] to [5].
[22] A pharmaceutical composition comprising: the nucleic acid according to [18], the expression vector according to [19], or the antibody according to [21].
[23] The pharmaceutical composition according to [19], which is for preventing, ameliorating, or treating a neurodegenerative disease.
[24] Use of the polypeptide according to any one of [1] to [5] or the conjugate according to any one of [6] to [12] in production of a vaccine for preventing, ameliorating, or treating a neurodegenerative disease.
[25] A method for preventing, ameliorating, or treating a neurodegenerative disease, the method comprising administering an effective amount of the polypeptide according to any one of [1] to [5] or the conjugate according to any one of [6] to [12] to a subject in need thereof.
[26] Use of the nucleic acid according to [18], the expression vector according to [19], the recombinant cell according to [20], or the antibody according to [21] in production of a pharmaceutical composition for preventing, ameliorating, or treating a neurodegenerative disease.
[27] A method for preventing, ameliorating, or treating a neurodegenerative disease, the method comprising administering an effective amount of the nucleic acid according to [18], the expression vector according to [19], or the antibody according to [21] to a subject in need thereof.
[28] The polypeptide according to any one of [1] to [5], the conjugate according to any one of [6] to [12], the nucleic acid according to [18], the expression vector according to [19], or the antibody according to [21] for use in prevention, amelioration, or treatment of a neurodegenerative disease.

### Advantageous Effects of Invention

According to the polypeptide of the present invention, neutralizing antibodies not only to α-synuclein monomers but also to α-synuclein oligomers can be induced. Therefore, therapeutic effects on neurodegenerative diseases comprising Parkinson's disease can be expected.

### Brief Description of Drawings

FIG. 1A shows the results of evaluating the phosphorylation of Syn due to aggregated Syn using SH-SY5Y cells in Example 5.
FIG. 1B shows the results of evaluating the human αSyn-neutralizing effect of an antibody induced in mice by an AJ-human αSyn peptide conjugate in Example 5.
FIG. 2A shows the results of evaluating, by a dot blot, the inhibitory effect of the AJ-human αSyn peptide conjugate on human αSyn propagation to the cortex in human αSyn-overexpressing mice in Example 6.
FIG. 2B shows the results of evaluating, by the dot blot, the inhibitory effect of the AJ-human αSyn peptide conjugate on human αSyn propagation to the olfactory bulb in the human αSyn-overexpressing mice in Example 6.
FIG. 3A shows the results of evaluating, by a western blot, the inhibitory effect of the AJ-human αSyn peptide conjugate on human αSyn propagation to the cortex in the human αSyn-overexpressing mice in Example 6.
FIG. 3B shows the results of evaluating, by the western blot, the inhibitory effect of the AJ-human αSyn peptide conjugate on human αSyn propagation to the olfactory bulb in the human αSyn-overexpressing mice in Example 6.
FIG. 4A shows the results of evaluating the ability of the AJ-human αSyn peptide conjugate to induce an anti-αSyn monomer antibody in cynomolgus monkeys in Example 7.
FIG. 4B shows the results of evaluating the ability of the AJ-human αSyn peptide conjugate to induce an anti-αSyn oligomer antibody in the cynomolgus monkeys in Example 7.
FIG. 5 shows the results of evaluating the human αSyn-neutralizing effect of an antibody induced in cynomolgus monkeys by the AJ-human αSyn peptide conjugate in Example 8.

### Description of Embodiments

### [Polypeptide]

Human α-synuclein (αSyn) is a protein that is encoded by a SNCA gene (Genbank No: 6622, Gene Accession No: NP_000336.1, Protein Accession No: P37840), consists of 140 amino acids set forth in SEQ ID NO: 14, and has a molecular weight of 14.5 kDa. It is known that an aggregate (oligomer) in which αSyn is highly phosphorylated and abnormally aggregated is a major constituent component of Lewy bodies, and that synucleinopathies are neurodegenerative diseases characterized by the abnormal accumulation of the aggregates of αSyn. As opposed to the aggregate (oligomer), non-aggregated αSyn is called a monomer of αSyn.

A polypeptide of one embodiment consists of any of the following amino acid sequences:
(a) an amino acid sequence set forth in SEQ ID NO: 1;
(b) an amino acid sequence set forth in SEQ ID NO: 2; and
(c) an amino acid sequence in which one, two, or three amino acids are substituted, deleted, added, and/or inserted in the amino acid sequence set forth in SEQ ID NO: 1 or SEQ ID NO: 2.

The polypeptide consisting of the amino acid sequence (KKDQLGKNE) set forth in SEQ ID NO: 1 is a polypeptide consisting of 96th to 104th amino acids (aa) of human αSyn set forth in SEQ ID NO: 14, and is referred to as p54 in the present specification. In addition, the polypeptide consisting of the amino acid sequence (EAYEMPSEE) set forth in SEQ ID NO: 2 is a polypeptide consisting of 123rd to 131st amino acids of human αSyn set forth in SEQ ID NO: 14, and is referred to as p150 in the present specification. As demonstrated in the Examples, p54 and p150 are excellent neutralizing antibody-inducing epitopes and can also induce neutralizing antibodies not only to α-synuclein monomers, but also to α-synuclein oligomers. Therefore, p54 and p150 can serve as vaccines for preventing, ameliorating, or treating diseases associated with α-synuclein accumulation.

In addition to the two epitopes (antigenic determinants), which are p54 and p150, a polypeptide (mimic) which mimics the structure of these epitopes known as a "mimotope" can also serve as a useful antigen. Such a mimic (mimotope) may be a polypeptide consisting of the amino acid sequence described in (c) above.

The substitution, deletion, addition, and/or insertion of one, two, or three amino acids means the substitution, deletion, addition, and/or insertion of one, two, or three amino acids at an arbitrary position in the amino acid sequence set forth in SEQ ID NO: 1 or SEQ ID NO: 2, or means the substitution, deletion, addition, and/or insertion of an arbitrary amino acid.

An amino acid that is substituted, inserted, or added may be natural or unnatural. Examples of natural amino acids comprise L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine. Examples of unnatural amino acids comprise D-form amino acids of the above-mentioned natural amino acids, and derivatives of natural amino acids in which introduction of various substituents into natural amino acids and the like have been performed.

The substitution of amino acids is not particularly limited, and examples of mutually substitutable amino acids having similar chemical properties are shown below as examples. Amino acids comprised in the same group are mutually substitutable (so-called conservative substitution), but amino acids at positions that have less influence on epitope properties may be substituted with amino acids comprised in different groups.
Group 1: leucine (L), isoleucine (I), norleucine, valine (V), norvaline, alanine (A), 2-aminobutanoic acid, methionine (M), o-methylserine, t-butylglycine, t-butylalanine, and cyclohexylalanine
Group 2: aspartic acid (D), glutamic acid (E), isoaspartic acid, isoglutamic acid, 2-aminoadipic acid, and 2-aminosuberic acid
Group 3: asparagine (N) and glutamine (Q)
Group 4: lysine (K), arginine (R), ornithine, 2,4-diaminobutanoic acid, and 2,3-diaminopropionic acid
Group 5: proline (P), 3-hydroxyproline, and 4-hydroxyproline
Group 6: serine (S), threonine (T), and homoserine
Group 7: phenylalanine (F) and tyrosine (Y)

The substitution or insertion of one, two, or three amino acids in the amino acid sequence set forth in SEQ ID NO: 1 may be the substitution or insertion of one, two, or three amino acids at an arbitrary position in the 96th, 97th, 98th, 99th, 100th, 101st, 102nd, 103rd, and 104th of SEQ ID NO: 14, or may the substitution of one, two, or three amino acids at an arbitrary position in the 97th, 98th, 99th, 102nd, 103rd, and 104th of SEQ ID NO: 14, for example. The deletion of one, two, or three amino acids in the amino acid sequence set forth in SEQ ID NO: 1 may be the deletion of one, two, or three amino acids at an arbitrary position in the 96th, 97th, 98th, 99th, 100th, 101st, 102nd, 103rd, and 104th of SEQ ID NO: 14. The addition of one, two, or three amino acids in the amino acid sequence set forth in SEQ ID NO: 1 may be the addition of one, two, or three amino acids to the N-terminal or C-terminal of the polypeptide having the amino acid sequence set forth in SEQ ID NO: 1. For example, the 95th amino acid, two amino acids from 94th and 95th, or three amino acids from 93rd to 95th of SEQ ID NO: 14 may be added to the N-terminal of the polypeptide having the amino acid sequence set forth in SEQ ID NO: 1, and the 105th amino acid, two amino acids from 105th and 106th, or three amino acids from 105th to 107th of SEQ ID NO: 14 may be added to the C-terminal of the polypeptide having the amino acid sequence set forth in SEQ ID NO: 1. Amino acids may be added to each of both the N-terminal and the C-terminal of the polypeptide having the amino acid sequence set forth in SEQ ID NO: 1. For example, the 95th amino acid of SEQ ID NO: 14 may be added to the N-terminal, and the 105th amino acid and the 106th amino acid may be added to the C-terminal; the 94th amino acid and the 95th amino acid of SEQ ID NO: 14 may be added to the N-terminal, and the 105th amino acid may be added to the C-terminal; and the 95th amino acid of SEQ ID NO: 14 may be added to the N-terminal, and the 105th amino acid may be added to the C-terminal. The combination of substitution, deletion, addition, or insertion may be arbitrary.

The substitution or insertion of one, two, or three amino acids in the amino acid sequence set forth in SEQ ID NO: 2 may be the substitution or insertion of one, two, or three arbitrary amino acids at an arbitrary position in the 123rd, 124th, 125th, 126th, 127th, 128th, 129th, 130th, and 131st of SEQ ID NO: 14, or may the substitution to arbitrary amino acids which is the substitution of one, two, or three amino acids at an arbitrary position in the 123rd, 125th, and 131st of SEQ ID NO: 14, for example. The deletion of one, two, or three amino acids in the amino acid sequence set forth in SEQ ID NO: 2 may be the deletion of one, two, or three amino acids at an arbitrary position in the 123rd, 124th, 125th, 126th, 127th, 128th, 129th, 130th, and 131st of SEQ ID NO: 14. The addition of one, two, or three amino acids in the amino acid sequence set forth in SEQ ID NO: 2 may be the addition of one, two, or three arbitrary amino acids to the N-terminal or C-terminal of the polypeptide having the amino acid sequence set forth in SEQ ID NO: 2. For example, the 122nd amino acid, two amino acids from 121st and 122nd, or three amino acids from 120th to 122nd of SEQ ID NO: 14 may be added to the N-terminal of the polypeptide having the amino acid sequence set forth in SEQ ID NO: 2, and the 132nd amino acid, two amino acids from 132nd and 133rd, or three amino acids from 132nd to 134th of SEQ ID NO: 14 may be added to the C-terminal of the polypeptide having the amino acid sequence set forth in SEQ ID NO: 2. Amino acids may be added to each of both the N-terminal and the C-terminal of the polypeptide having the amino acid sequence set forth in SEQ ID NO: 2. For example, the 122nd amino acid of SEQ ID NO: 14 may be added to the N-terminal, and the 132nd amino acid and the 133rd amino acid may be added to the C-terminal; the 121st amino acid and the 122nd amino acid of SEQ ID NO: 14 may be added to the N-terminal, and the 132nd amino acid may be added to the C-terminal; and the 122nd amino acid of SEQ ID NO: 14 may be added to the N-terminal, and the 132nd amino acid may be added to the C-terminal. The combination of substitution, deletion, addition, or insertion may be arbitrary.

Specific examples of the polypeptide consisting of the amino acid sequence described in (c) above comprise a polypeptide consisting of an amino acid sequence set forth in any one of SEQ ID NOs: 4 to 9 and SEQ ID NOs: 22 to 24 below which are amino acid sequence in which one, two, or three amino acids have been substituted, deleted, added, and/or inserted in the amino acid sequence set forth in SEQ ID NO: 1; and a polypeptide consisting of an amino acid sequence set forth in any one of SEQ ID NO: 3, SEQ ID NO: 10, SEQ ID NO: 11, and SEQ ID NOs: 18 to 21 which are amino acid sequence in which one, two, or three amino acids have been substituted, deleted, added, and/or inserted in the amino acid sequence set forth in SEQ ID NO: 2. In other words, examples thereof comprise p283, p142, p167, p227, p233, p272, p273, p276, p277, p242, p244, p347, p361, p229, p237, and p298 below. Among them, p167, p227, p272, p276, p283, p242, p244, and p229 are particularly preferably used because of a high neutralizing antibody-inducing ability with respect to α-synuclein oligomers.

p283 (123-132aa of SEQ ID NO: 14, E131D): EAYEMPSEDG (SEQ ID NO: 3). The phrase "123-132aa of SEQ ID NO: 14, E131D" means that the 131st glutamic acid (E) has been substituted with aspartic acid (D) in the 123rd to 132nd sequences of the amino acid sequence set forth in SEQ ID NO: 14. In the amino acid sequence of p283, the 9th glutamic acid (E) of the amino acid sequence set forth in SEQ ID NO: 2 has been substituted with aspartic acid (D), and the 132nd glycine (G) of the amino acid sequence set forth in SEQ ID NO: 14 has been added as the 10th amino acid to the C-terminal of the amino acid sequence set forth in SEQ ID NO: 2.

p142 (96-104aa of SEQ ID NO: 14, K102A): KKDQLGANE (SEQ ID NO: 4). The phrase "96-104aa of SEQ ID NO: 14, K102A" means that the 102nd lysine (K) has been substituted with alanine (A) in the 96th to 104th sequences of the amino acid sequence set forth in SEQ ID NO: 14. In the amino acid sequence of p142, the 7th lysine (K) of the amino acid sequence set forth in SEQ ID NO: 1 has been substituted with alanine (A).

p167 (96-105aa of SEQ ID NO: 14): KKDQLGKNEE (SEQ ID NO: 5). The phrase "96-105aa of SEQ ID NO: 14" means corresponding to the 96th to 105th sequences of the amino acid sequence set forth in SEQ ID NO: 14. In the amino acid sequence of p167, the 105th glutamic acid (E) of the amino acid sequence set forth in SEQ ID NO: 14 has been added as the 10th amino acid to the C-terminal of the amino acid sequence set forth in SEQ ID NO: 1.

p227 (96-104aa of SEQ ID NO: 14, K97R): KRDQLGKNE (SEQ ID NO: 6). The phrase "96-104aa of SEQ ID NO: 14, K97R" means that the 97th lysine (K) has been substituted with arginine (R) in the 96th to 104th sequences of the amino acid sequence set forth in SEQ ID NO: 14. In the amino acid sequence of p227, the 2nd lysine (K) of the amino acid sequence set forth in SEQ ID NO: 1 has been substituted with arginine (R).

p233 (96-104aa of SEQ ID NO: 14, E104D): KKDQLGKND (SEQ ID NO: 7). The phrase "96-104aa of SEQ ID NO: 14, E104D" means that the 104th glutamic acid (E) has been substituted with aspartic acid (D) in the 96th to 104th sequences of the amino acid sequence set forth in SEQ ID NO: 14. In the amino acid sequence of p227, the 9th glutamic acid (E) of the amino acid sequence set forth in SEQ ID NO: 1 has been substituted with aspartic acid (D).

p272 (96-104aa of SEQ ID NO: 14, D98N, E104D): KKNQLGKND (SEQ ID NO: 8). The phrase "96-104aa of SEQ ID NO: 14, D98N, E104D" means that the 98th aspartic acid (D) has been substituted with asparagine (N) and the 104th glutamic acid (E) has been substituted with aspartic acid (D) in the 96th to 104th sequences of the amino acid sequence set forth in SEQ ID NO: 14. In the amino acid sequence of p272, the 3rd aspartic acid (D) of the amino acid sequence set forth in SEQ ID NO: 1 has been substituted with asparagine (N), and the 9th glutamic acid (E) has been substituted with aspartic acid (D).

p273 (96-104aa of SEQ ID NO: 14, D98T, E104D): KKTQLGKND (SEQ ID NO: 9). The phrase "96-104aa of SEQ ID NO: 14, D98T, E104D" means that the 98th aspartic acid (D) has been substituted with threonine (T) and the 104th glutamic acid (E) has been substituted with aspartic acid (D) in the 96th to 104th sequences of the amino acid sequence set forth in SEQ ID NO: 14. In the amino acid sequence of p273, the 3rd aspartic acid (D) of the amino acid sequence set forth in SEQ ID NO: 1 has been substituted with threonine (T), and the 9th glutamic acid (E) has been substituted with aspartic acid (D).

p276 (123-132aa of SEQ ID NO: 14, E123D): DAYEMPSEEG (SEQ ID NO: 10). The phrase "123-132aa of SEQ ID NO: 14, E123D" means that the 123rd glutamic acid (E) has been substituted with aspartic acid (D) in the 123rd to 132nd sequences of the amino acid sequence set forth in SEQ ID NO: 14. In the amino acid sequence of p276, the 1st glutamic acid (E) of the amino acid sequence set forth in SEQ ID NO: 2 has been substituted with aspartic acid (D), and the 132nd glycine (G) of the amino acid sequence set forth in SEQ ID NO: 14 has been added as the 10th amino acid to the C-terminal of the amino acid sequence set forth in SEQ ID NO: 2.

p277 (123-132aa of SEQ ID NO: 14, Y125F): EAFEMPSEEG (SEQ ID NO: 11). The phrase "123-132aa of SEQ ID NO: 14, Y125F" means that the 125th tyrosine (Y) has been substituted with phenylalanine (F) in the 123rd to 132nd sequences of the amino acid sequence set forth in SEQ ID NO: 14. In the amino acid sequence of p277, the 3rd tyrosine (Y) of the amino acid sequence set forth in SEQ ID NO: 2 has been substituted with phenylalanine (F), and the 132nd glycine (G) of the amino acid sequence set forth in SEQ ID NO: 14 has been added as the 10th amino acid to the C-terminal of the amino acid sequence set forth in SEQ ID NO: 2.

p242 (123-132aa of SEQ ID NO: 14, E123A): AAYEMPSEEG (SEQ ID NO: 18). The phrase "123-132aa of SEQ ID NO: 14, E123A" means that the 123rd glutamic acid (E) has been substituted with alanine (A) in the 123rd to 132nd sequences of the amino acid sequence set forth in SEQ ID NO: 14. In the amino acid sequence of p242, the 1st glutamic acid (E) of the amino acid sequence set forth in SEQ ID NO: 2 has been substituted with alanine (A), and the 132nd glycine (G) of the amino acid sequence set forth in SEQ ID NO: 14 has been added as the 10th amino acid to the C-terminal of the amino acid sequence set forth in SEQ ID NO: 2.

p244 (123-132aa of SEQ ID NO: 14, Y125A): EAAEMPSEEG (SEQ ID NO: 19). The phrase "123-132aa of SEQ ID NO: 14, Y125A" means that the 125th tyrosine (Y) has been substituted with alanine (A) in the 123rd to 132nd sequences of the amino acid sequence set forth in SEQ ID NO: 14. In the amino acid sequence of p244, the 3rd tyrosine (Y) of the amino acid sequence set forth in SEQ ID NO: 2 has been substituted with alanine (A), and the 132nd glycine (G) of the amino acid sequence set forth in SEQ ID NO: 14 has been added as the 10th amino acid to the C-terminal of the amino acid sequence set forth in SEQ ID NO: 2.

p347 (123-132aa of SEQ ID NO: 14, E123H, Y125F): HAFEMPSEEG (SEQ ID NO: 20). The phrase "123-132aa of SEQ ID NO: 14, E123H, Y125F" means that the 123rd glutamic acid (E) has been substituted with histidine (H) and the 125th tyrosine (Y) has been substituted with phenylalanine (F) in the 123rd to 132nd sequences of the amino acid sequence set forth in SEQ ID NO: 14. In the amino acid sequence of p347, the 1st glutamic acid (E) of the amino acid sequence set forth in SEQ ID NO: 2 has been substituted with histidine (H), the 3rd tyrosine (Y) has been substituted with phenylalanine (F), and the 132nd glycine (G) of the amino acid sequence set forth in SEQ ID NO: 14 has been added as the 10th amino acid to the C-terminal of the amino acid sequence set forth in SEQ ID NO: 2.

p361 (123-132aa of SEQ ID NO: 14, E123D, Y125F): DAFEMPSEEG (SEQ ID NO: 21). The phrase "123-132aa of SEQ ID NO: 14, E123D, Y125F" means that the 123rd glutamic acid (E) has been substituted with aspartic acid (D) and the 125th tyrosine (Y) has been substituted with phenylalanine (F) in the 123rd to 132nd sequences of the amino acid sequence set forth in SEQ ID NO: 14. In the amino acid sequence of p361, the 1st glutamic acid (E) of the amino acid sequence set forth in SEQ ID NO: 2 has been substituted with aspartic acid (D), the 3rd tyrosine (Y) has been substituted with phenylalanine (F), and the 132nd glycine (G) of the amino acid sequence set forth in SEQ ID NO: 14 has been added as the 10th amino acid to the C-terminal of the amino acid sequence set forth in SEQ ID NO: 2.

p229 (96-104aa of SEQ ID NO: 14, D98T): KKTQLGKNE (SEQ ID NO: 22). The phrase "96-104aa of SEQ ID NO: 14, D98T" means that the 98th aspartic acid (D) has been substituted with threonine (T) in the 96th to 104th sequences of the amino acid sequence set forth in SEQ ID NO: 14. In the amino acid sequence of p229, the 3rd aspartic acid (D) of the amino acid sequence set forth in SEQ ID NO: 1 has been substituted with threonine (T).

p237 (96-104aa of SEQ ID NO: 14, D98N): KKNQLGKNE (SEQ ID NO: 23). The phrase "96-104aa of SEQ ID NO: 14, D98N" means that the 98th aspartic acid (D) has been substituted with asparagine (N) in the 96th to 104th sequences of the amino acid sequence set forth in SEQ ID NO: 14. In the amino acid sequence of p237, the 3rd aspartic acid (D) of the amino acid sequence set forth in SEQ ID NO: 1 has been substituted with asparagine (N).

p298 (96-104aa of SEQ ID NO: 14, Q99E, N103D): KKDELGKDE (SEQ ID NO: 24). The phrase "96-104aa of SEQ ID NO: 14, Q99E, N103D" means that the 99th glutamine (Q) has been substituted with glutamic acid (E) and the 103rd asparagine (N) has been substituted with aspartic acid (D) in the 96th to 104th sequences of the amino acid sequence set forth in SEQ ID NO: 14. In the amino acid sequence of p298, the 4th glutamine (Q) of the amino acid sequence set forth in SEQ ID NO: 1 has been substituted with glutamic acid (E), and the 8th asparagine (N) has been substituted with aspartic acid (D).

The polypeptide of the present embodiment can be produced by genetic engineering techniques. A production method is not particularly limited, and for example, the polypeptide consisting of the amino acid sequence set forth in SEQ ID NO: 1 or 2 can be obtained by designing a primer using the cDNA of a SNCA gene encoding human α-synuclein (αSyn) as a template to obtain a nucleic acid encoding a desired polypeptide by PCR, and functionally linking this nucleic acid to an expression promoter, which is then introduced into an appropriate expression vector, which is then expressed in a host cell, while also linking a nucleic acid encoding a carrier protein and a nucleic acid encoding a tag depending on cases. Furthermore, in the case of a mimotope, a nucleic acid encoding a polypeptide that is a mimotope can be obtained by introducing mutations by site-directed mutagenesis using the cDNA of a SNCA gene as a template, and substituting with a base sequence encoding another amino acid residue. Furthermore, the production is also possible by designing and artificially synthesizing a nucleic acid encoding a desired amino acid sequence without using a template. The nucleic acid herein may be DNA or RNA.

Meanwhile, the polypeptide of the present embodiment is a short peptide, and thus can also be produced by artificial synthesis according to a desired amino acid sequence. An artificial synthesis method is not particularly limited as long as it is a known method, and examples thereof comprise solid-phase synthesis methods such as a Fmoc method and a Boc method, and liquid-phase synthesis methods.

In addition, the produced polypeptide may be purified as necessary. A purification method is not particularly limited, and examples thereof comprise purification using an affinity chromatography column or the like.

### [Conjugate]

A conjugate of one embodiment comprises the polypeptide of the above-mentioned embodiment and a carrier protein. In the conjugate, the polypeptide and the carrier protein are chemically linked. When a sufficient immune response cannot be induced because the polypeptide that is an antigen has a small molecular weight, the apparent molecular weight can be increased by linking the carrier protein, which makes it possible to enhance an antibody-inducing ability.

The carrier protein is not particularly limited as long as it can improve the antigenicity of an antigenic peptide, and examples thereof comprise common carrier proteins such as bovine serum albumin (BSA), keyhole limpet hemocyanin (KLH), and ovalbumin (OVA). Furthermore, a peptide (referred to as "AJ peptide" in the present specification) consisting of an amino acid sequence identical or substantially identical to an amino acid sequence set forth in SEQ ID NO: 13 (ELKLIFLHRLKRLRKRLKRK) is also preferably used. Because the AJ peptide itself has low antigenicity, unfavorable effects resulting from the antigenicity of the carrier protein can be reduced. In addition, although the AJ peptide is a short peptide, it has a strong adjuvant effect (Patent Literature 4).

Examples of the amino acid sequence substantially identical to the amino acid sequence set forth in SEQ ID NO: 13 comprise an amino acid sequence in which one to four amino acids have been substituted, deleted, added, and/or inserted in the amino acid sequence set forth in SEQ ID NO: 13, among which an amino acid sequence in which one to three amino acids have been substituted, deleted, added, and/or inserted is preferable, an amino acid sequence in which one or two amino acids have been substituted, deleted, added, and/or inserted is more preferable, and an amino acid sequence in which one amino acid has been substituted, deleted, added, and/or inserted is further preferable. Alternatively, the substantially identical amino acid sequence refers to an amino acid sequence that is 80% or more homologous to the amino acid sequence set forth in SEQ ID NO: 13, and an amino acid sequence that is preferably 85% or more, more preferably 90% or more, and further preferably 95% or more homologous thereto.

The peptide consisting of the amino acid sequence substantially identical to the amino acid sequence set forth in SEQ ID NO: 13 is preferably the peptide having substantially the same activity as the peptide consisting of the amino acid sequence set forth in SEQ ID NO: 13. Specifically, the antibody-inducing ability of a conjugate of an epitope and the peptide consisting of the amino acid sequence substantially identical to the peptide consisting of the amino acid sequence set forth in SEQ ID NO: 13 is preferably equal to or greater than the antibody-inducing ability of a conjugate of an epitope and the peptide consisting of the amino acid sequence set forth in SEQ ID NO: 13. For evaluation of whether or not the antibody-inducing ability is equal or greater, evaluation can be performed by immunizing animals in the same operation using a conjugate obtained by similarly linking the peptide consisting of the amino acid sequence set forth in SEQ ID NO: 13 (reference peptide) or a peptide to be evaluated to the same antigen epitope, and measuring and comparing the antibody titers of the obtained immune serum by ELISA. When the antibody titer of the conjugate to which the peptide to be evaluated has been linked is 80% or more, preferably 90% or more, and more preferably 95% or more of the antibody titer of the conjugate to which the reference peptide has been linked, this can be evaluated that the peptide to be evaluated has an antibody-inducing ability that is equal to or greater than that of the reference peptide. The antibody titer may be measured by a common method such as a method described in the Examples, for example.

The polypeptide and the carrier protein may be linked in the order of the polypeptide and the carrier protein from the N-terminal side, or may be linked in the order of the carrier protein and the polypeptide. Furthermore, there may be one type of the carrier protein, or two types thereof. In the case of the two types, linkage may be performed in the order of the carrier protein of the first type, the polypeptide, and the carrier protein of the second type from the N-terminal side. In addition, for the purpose of improving stability and for the purpose of improving cell permeability by removal of charge, an N-terminal amino acid of the conjugate is preferably acetylated, and a C-terminal amino acid is preferably amidated.

The polypeptide and the carrier protein may be fused via a peptide linker or directly, or alternatively, may be chemically linked via a linker, or alternatively, may be linked via both a linker in a chemical linkage and a peptide linker. The peptide linker is not particularly limited, and examples thereof comprise a linker consisting of one to 50 amino acids, preferably one to 20 amino acids, or a linker consisting of 15 to 20 amino acids, for example, a peptide linker consisting of one to five or one to six naturally occurring L-amino acids or a repeating sequence thereof. Specifically, a linker consisting of Gly-Gly-Gly-Gly-Ser (GGGGS; SEQ ID NO: 15) or a repeating sequence thereof, a linker consisting of Glu-Ala-Ala-Ala-Lys (EAAAK; SEQ ID NO: 16) or a repeating sequence thereof, a linker consisting of Asp-Asp-Ala-Lys-Lys (DDAKK; SEQ ID NO: 17) or a repeating sequence thereof, a linker consisting of Lys-Lys-Lys (KKK) (preferably εKKK) or a repeating sequence thereof, a linker consisting of Lys-Lys-Lys-Lys (KKKK; SEQ ID NO: 25) (preferably εKKKK) or a repeating sequence thereof, and a linker consisting of Pro-Pro-Asp-Pro-Asp-Pro (PPDPDP; SEQ ID NO: 26) or a repeating sequence thereof. These amino acids may be natural L-amino acids or may be D-amino acids. In addition, the linker in a chemical linkage is not particularly limited, and examples thereof comprise ε-aminocaproic acid, β-aminoalanine, γ-aminobutyric acid, 7-aminoheptanoic acid, 12-aminolauric acid, glutamic acid, and p-aminobenzoic acid. The AJ peptide and the above-mentioned polypeptide may be linked via ε-aminocaproic acid, and KLH and the above-mentioned polypeptide may be linked via a peptide linker such as cysteine. The AJ peptide and the above-mentioned polypeptide may be linked via ε-aminocaproic acid and/or the peptide linker.

Specific examples of the conjugates comprise AJ-p54, AJ-p150, AJ-p283, AJ-p54-KLH, KLH-AJ-p54, KLH-p54, p54-KLH, AJ-p142, AJ-p167, AJ-p227, AJ-p233, AJ-p272, AJ-p273, AJ-p276, and AJ-p277, which are shown in Table 1; and AJ-p242, AJ-p244, AJ-p347, AJ-p361, AJ-p229, AJ-p237, and AJ-p298, which are shown in Table 5. Among them, AJ-p54, AJ-p150, AJ-p167, AJ-p227, AJ-p272, AJ-p276, AJ-p283, AJ-p242, AJ-p244, and AJ-p229 are particularly preferable because they have a high neutralizing antibody-inducing ability with respect to α-synuclein oligomers.

The conjugate can be produced by methods known to those skilled in the art. When the conjugate is a fusion protein, the conjugate that is a fusion protein can be obtained by producing a nucleic acid encoding a fusion protein in which the polypeptide and the carrier protein are functionally linked, optionally via the peptide linker, by a PCR method as described above, and functionally linking this nucleic acid to an expression promoter, which is then introduced into an appropriate expression vector, which is then expressed in a host cell. In addition, when a fusion protein has a short amino acid sequence, the production is also possible by artificial synthesis by a known peptide synthesis method (such as a solid-phase synthesis method).

The conjugate linked via a chemical linker can be produced by separately producing the peptide and carrier protein and then linking them. For example, the obtainment is possible by producing each of the polypeptide that is an epitope, and the carrier protein, linking via a chemical linker (such as ε-aminocaproic acid) after adding a protecting group, and performing condensation and deprotection. Examples of the protecting groups comprise a tert-Butyl Oxy Carbonyl (tBOC) group, and a Fluorenyl-MethOxy-Carbonyl (FMOC) group. As condensing agents, for example, it is possible to use carbodiimide compounds such as DCC (N,N'-Dicyclohexylcarbodiimide) and EDC ((N-(3-Dimethylaminopropyl)-N'-ethylcarbodiimide); imidazole compounds such as CDI (1,1'-Carbonyldiimidazole); phosphonium salt compounds such as BOP (benzotriazol-1-yloxytris(dimethylamino)phosphonium hexafluorophosphate); uronium salt compounds such as HBTU (O-(Benzotriazol-1-yl)-N,N,N',N'-tetramethyluronium hexafluorophosphate) and HCTU (O-(6-Chlorobenzotriazol-1-yl)-N,N,N',N'-tetramethyluronium hexafluorophosphate); and the like.

### [Antibody]

One embodiment provides an antibody that specifically binds to a polypeptide that is an epitope (comprising a mimotope) of human α-synuclein (αSyn). The antibody of the present embodiment is used for preventing, ameliorating, or treating neurodegenerative diseases.

The neurodegenerative diseases may be diseases associated with the accumulation of α-synuclein. The accumulation of α-synuclein means the accumulation of especially α-synuclein aggregates (also called "aggregated α-synuclein" or "α-synuclein oligomer") in nerve cells, nerve fibers, or glial cells. Examples of the diseases associated with the accumulation of α-synuclein comprise synucleinopathies such as Parkinson's disease (PD), dementia with Lewy bodies (DLB), multiple system atrophy (MSA), pure autonomic failure (PAF), and neurodegeneration with brain iron accumulation (NBIA), and particularly comprise Parkinson's disease. Therefore, the antibody of the present embodiment is preferably capable of specifically binding not only to αSyn monomers but also to aggregated α-synuclein.

The antibody can be obtained from immune serum by immunizing mammals with the above-mentioned polypeptide or the above-mentioned conjugate, or with the following pharmaceutical composition (vaccine) by known methods. In immunization, a dosage and a dosing interval may be as described in [Pharmaceutical composition] to be described later. Examples of the mammals comprise mammals such as humans, Chinese hamsters, mice, rats, rabbits, dogs, pigs, monkeys, goats, and horses. Therefore, the antibody may be an antibody derived from these mammals, and is particularly preferably a human antibody (human-type antibody). In addition, the antibody may be a chimeric antibody or a humanized antibody. The chimeric antibody may have a variable region from one animal species and a constant region from another animal species, for example. For example, the chimeric antibody may be an antibody (humanized antibody) having a variable region derived from a mouse monoclonal antibody and a constant region derived from a human antibody.

The antibody may be a monoclonal antibody or a polyclonal antibody, but is preferably a monoclonal antibody. In addition, the antibody may be a full-length antibody (natural antibody) or may be an antigen-binding fragment. The natural antibody can be selected from the group consisting of IgG, IgM, IgA, IgD, and IgE. Examples of the antigen-binding fragments comprise (i) a Fab fragment that is a monovalent fragment consisting of V_{L}, V_{H}, C_{L}, and C_{H1} domains; (ii) a F(ab')₂ fragment that is a bivalent fragment comprising two Fab fragments linked by a disulfide bridge at a hinge region; (iii) a Fd fragment consisting of V_{H} and C_{H1} domains; (iv) a Fv fragment consisting of V_{L} and V_{H} of a one-armed antibody; and (v) a dAb fragment consisting of a V_{H} domain.

The antibody of the present embodiment is administered in an effective amount for preventing, ameliorating, or treating the neurodegenerative diseases. An administration subject may be a mammal such as a human, but is particularly preferably a human. The dosage and the dosing interval vary depending on the half-life of the antibody in the subject. For example, the dosage may be 0.0001 mg/kg to 100 mg/kg body weight, and may be 0.01 mg/kg to 10 mg/kg body weight, or 0.1 to 10 mg/kg, for example. The dosing interval is not particularly limited, and may be once every two weeks, once a month, once every three to six months, once a year, or once every two to five years. The dosing interval may be an irregular dosing interval in which the concentration of an anti-α-synuclein antibody in the plasma of the subject is monitored to perform administration when the concentration falls below a certain threshold value (for example, a plasma antibody concentration of 1 to 1000 µg/ml). Alternatively, one type of antibody may be administered, or two or more types of monoclonal antibodies having different binding specificities may be administered simultaneously or alternately. An administration route is not particularly limited, and examples thereof comprise oral, parenteral, topical, intravenous, subcutaneous, intraarterial, intracranial, subarachnoid, intraperitoneal, intranasal, and intramuscular routes.

### [Nucleic acid, expression vector, and recombinant cell]

One embodiment provides a nucleic acid encoding the above-mentioned polypeptide or the above-mentioned conjugate that is a fusion protein. The nucleic acid of the present embodiment is used for preventing, ameliorating, or treating neurodegenerative diseases. The neurodegenerative diseases are as described above.

The nucleic acid of the present embodiment is a nucleic acid encoding the above-mentioned polypeptide or the above-mentioned conjugate that is a fusion protein, and may be DNA or RNA. The nucleic acid may be produced by PCR as described above, or may be artificially synthesized.

An expression vector of one embodiment comprises the nucleic acid encoding the above-mentioned polypeptide or the above-mentioned conjugate that is a fusion protein. The expression vector is not particularly limited as long as it is a vector capable of autonomous replication in a host cell and/or a vector capable of integration into the chromosome of a host cell, and is a vector capable of transcribing and expressing the above-mentioned nucleic acid. When prokaryotes such as bacteria are used as host cells, the expression vector preferably further comprises a promoter, a ribosome binding sequence, and a transcription termination sequence in addition to the above-mentioned nucleic acids, and may further comprise a gene that controls the promoter. Such a vector may be a known vector, and a suitable vector can be selected by those skilled in the art depending on host cells.

As the expression vector, retroviral systems, adenovirus vectors, adeno-associated virus vectors, virus vectors derived from the family *Poxviridae* comprising *vaccinia* virus and *Avipoxvirus,* virus vectors derived from the genus *Alphavirus,* Venezuelan equine encephalitis virus, rhabdovirus, and the like may be used.

The nucleic acid encoding the polypeptide or the conjugate that is a fusion protein, or the expression vector comprising the nucleic acid can be encapsulated in liposomes. The nucleic acid encoding the polypeptide or the conjugate that is a fusion protein, or the expression vector comprising the nucleic acid can also be adsorbed to or bound to a particulate carrier (such as a polymethyl methacrylate polymer).

An effective amount of the nucleic acid encoding the polypeptide or the conjugate that is a fusion protein, the expression vector comprising the nucleic acid, the liposome in which the nucleic acid or the expression vector is encapsulated, or the particulate carrier carrying the nucleic acid or the expression vector may be administered to the subject for preventing, ameliorating, or treating the neurodegenerative diseases. An administration route is not particularly limited, and examples thereof comprise oral, parenteral, topical, intravenous, subcutaneous, intraarterial, intracranial, subarachnoid, intraperitoneal, intranasal, and intramuscular routes. The dosage may be 0.0001 mg to 100 mg, and may be 0.001 mg to 10 mg, for example, in terms of the amount of the nucleic acid encoding the polypeptide or the conjugate that is a fusion protein, for example. The dosing interval is not particularly limited, and may be once every two weeks, once a month, once every three to six months, once a year, or once every two to five years. In the subject, the expression of the nucleic acid produces an epitope in the body, thereby inducing an antibody that specifically binds to the epitope. Therefore, the dosing interval may be an irregular dosing interval in which the concentration of an anti-α-synuclein antibody in the plasma of the subject is monitored to perform administration when the concentration falls below a certain threshold value (for example, a plasma antibody concentration of 1 to 1000 µg/ml).

A recombinant cell of one embodiment is a recombinant cell obtained by transforming a host cell with the above-mentioned expression vector. In the recombinant cell, the nucleic acid or the expression vector mentioned above may be integrated into the genome, or may be present as a plasmid capable of autonomous replication, but the above-mentioned nucleic acid is comprised in the state capable of being transcribed. Host cells may be any of prokaryotes, yeast, animal cells, insect cells, plant cells, and the like, but may be preferably prokaryotes or yeast strains. Examples thereof comprise prokaryotes such as *Escherichia coli,* and yeast strains such as *Saccharomyces cerevisiae.* The above-mentioned polypeptide or the above-mentioned conjugate that is a fusion protein can be produced using the recombinant cell of the present embodiment.

### [Pharmaceutical composition]

A pharmaceutical composition of one embodiment is a pharmaceutical composition comprising the polypeptide of the above-mentioned embodiment or the conjugate of the above-mentioned embodiment. The present pharmaceutical composition may be a pharmaceutical composition for preventing, ameliorating, or treating neurodegenerative diseases, and is particularly preferably a vaccine.

The dosage form of the pharmaceutical composition of the present embodiment may be a liquid, a powder (freeze-dried powder, dry powder), a capsule, a tablet, or a frozen state, for example.

The pharmaceutical composition of the present embodiment may comprise a pharmaceutically acceptable carrier. The above-mentioned carrier may be a carrier commonly used in vaccine production, and specific examples thereof comprise saline, buffered saline, dextrose, water, glycerol, isotonic aqueous buffer solutions, and combinations thereof. An emulsifier, a preservative (such as thimerosal), a tonicity agent, a pH adjuster, an inactivator (such as formalin), or the like may be further blended in the vaccine as appropriate.

An adjuvant can also be comprised to enhance the immunogenicity of the vaccine. Examples of the adjuvants comprise aluminum adjuvants or oil-in-water-type emulsion adjuvants comprising squalene (such as AS03 and MF59); toll-like receptor ligands such as CpG and 3-O-deacylated-4'-monophosphoryl lipid A (MPL); saponin-based adjuvants; polymer-based adjuvants such as poly γ-glutamic acid; and polysaccharides such as chitosan and inulin. Specific examples thereof comprise aluminum hydroxide (Alum) and Complete Freund's adjuvant (CFA) (DIFCO).

The pharmaceutical composition of the present embodiment can be produced by mixing the carrier, the adjuvant, and the like as necessary, in addition to the above-mentioned polypeptide or the above-mentioned conjugate. The adjuvant may be mixed at the time of use.

The administration subject of the pharmaceutical composition of the present embodiment is mammals, particularly humans. The administration route of the pharmaceutical composition of the present embodiment may be transdermal administration, sublingual administration, eye drop administration, intracutaneous administration, intramuscular administration, oral administration, enteral administration, nasal administration, intravenous administration, subcutaneous administration, intraperitoneal administration, or mouth-to-lung inhalation administration, for example.

A method of administering the pharmaceutical composition of the present embodiment may be a method of administering by syringes, transdermal patches, microneedles, implantable sustained-release devices, syringes with microneedles, needle-free devices, or sprays, for example.

The pharmaceutical composition of the present embodiment is a pharmaceutical composition for preventing, ameliorating, or treating neurodegenerative diseases, in which the neurodegenerative diseases may be diseases associated with the accumulation of α-synuclein. The accumulation of α-synuclein means the accumulation of especially α-synuclein aggregates (also called "aggregated α-synuclein" or "α-synuclein oligomer") in nerve cells, nerve fibers, or glial cells. Examples of the diseases associated with the accumulation of α-synuclein comprise synucleinopathies such as Parkinson's disease (PD), dementia with Lewy bodies (DLB), multiple system atrophy (MSA), pure autonomic failure (PAF), and neurodegeneration with brain iron accumulation (NBIA), and particularly comprise Parkinson's disease. As other neurodegenerative diseases, Alzheimer's disease (AD) caused by the deposition of tau and amyloid beta (Aβ) in the brain and amyotrophic lateral sclerosis (ALS) caused by the deposition of the aggregate of TDP-43 protein in the brain are also thought to be the diseases associated with the accumulation of α-synuclein because α-synuclein is correlated with the aggregation of these proteins.

The pharmaceutical composition of the present embodiment comprises an effective amount of the above-mentioned polypeptide or the above-mentioned conjugate, and preferably comprises the polypeptide in an amount of 0.0001 mg to 1,000 mg, preferably 0.001 mg to 1,000 mg, and more preferably 0.1 mg to 100 mg in terms of the amount of the above-mentioned polypeptide.

By administering the pharmaceutical composition of the present embodiment to the subject, an anti-human α-synuclein antibody is induced in the subject, and this antibody specifically binds not only to human α-synuclein monomers but also to human α-synuclein oligomers. Thereby, the neurodegenerative diseases caused by the aggregate of α-synuclein can be prevented, ameliorated, or treated.

The number of doses and the dosing interval of the pharmaceutical composition of the present embodiment are not particularly limited, but single administration may be performed, or multiple administrations may be performed at intervals of several days to several months. A single dosage of the pharmaceutical composition of the present embodiment can be appropriately adjusted depending on administration subjects, administration methods, and the like, but is 0.0001 mg to 1,000 mg, preferably 0.001 mg to 1,000 mg, and more preferably 0.1 mg to 100 mg. The efficacy of the pharmaceutical composition of the present embodiment can be evaluated by detecting an anti-human α-synuclein antibody in the serum of the administration subject.

A pharmaceutical composition of another embodiment is a pharmaceutical composition comprising the nucleic acid or the expression vector of the above-mentioned embodiment. The pharmaceutical composition of the present embodiment may be a pharmaceutical composition for preventing, ameliorating, or treating neurodegenerative diseases, and is particularly preferably a vaccine. The above-mentioned nucleic acid or the above-mentioned expression vector may be in the form of a liposome in which the above-mentioned nucleic acid or the above-mentioned expression vector is encapsulated, or a particulate carrier carrying the above-mentioned nucleic acid or the above-mentioned expression vector, and the effective amount (administration amount), the administration route, and the dosing interval thereof are as described in [Nucleic acid, expression vector, and recombinant cell] described above. The dosage form of the pharmaceutical composition of the present embodiment may be a liquid, a powder (freeze-dried powder, dry powder), a capsule, a tablet, or a frozen state, for example. The pharmaceutical composition of the present embodiment may comprise a pharmaceutically acceptable carrier.

A pharmaceutical composition of still another embodiment is a pharmaceutical composition comprising the antibody of the above-mentioned embodiment. The pharmaceutical composition of the present embodiment is a pharmaceutical composition for preventing, ameliorating, or treating neurodegenerative diseases, and the dosage, the administration route, the dosing interval, and the like are as described in [Antibody] described above. The dosage form of the pharmaceutical composition of the present embodiment may be a liquid, a powder (freeze-dried powder, dry powder), a capsule, a tablet, or a frozen state, for example. The pharmaceutical composition of the present embodiment may comprise a pharmaceutically acceptable carrier.

### [Use and method]

One embodiment of the present invention provides use of the polypeptide of the above-mentioned embodiment or the conjugate of the above-mentioned embodiment in the production of a vaccine for preventing, ameliorating, or treating neurodegenerative diseases.

One embodiment of the present invention provides a method for preventing, ameliorating, or treating neurodegenerative diseases, the method comprising administering an effective amount of the polypeptide of the above-mentioned embodiment or the conjugate of the above-mentioned embodiment to a subject in need thereof (such as a patient with a neurodegenerative disease or a subject in need of preventing a neurodegenerative disease).

One embodiment of the present invention provides use of the nucleic acid of the above-mentioned embodiment, the expression vector of the above-mentioned embodiment, the recombinant cell of the above-mentioned embodiment, or the antibody of the above-mentioned embodiment in the production of a pharmaceutical composition for preventing, ameliorating, or treating neurodegenerative diseases.

One embodiment of the present invention provides a method for preventing, ameliorating, or treating neurodegenerative diseases, the method comprising administering an effective amount of the nucleic acid of the above-mentioned embodiment, the expression vector of the above-mentioned embodiment, or the antibody of the above-mentioned embodiment to a subject in need thereof (such as a patient with a neurodegenerative disease or a subject in need of preventing a neurodegenerative disease).

One embodiment of the present invention provides the polypeptide of the above-mentioned embodiment, the conjugate of the above-mentioned embodiment, the nucleic acid of the above-mentioned embodiment, the expression vector of the above-mentioned embodiment, or the antibody of the above-mentioned embodiment for preventing, ameliorating, or treating neurodegenerative diseases.

The present invention will be described in detail below with reference to examples, but the present invention is not limited to these.

### [Examples]

### Example 1 Production of various epitope peptides of human αSyn and conjugates

By a solid-phase synthesis method reported by R. B. Merrifield et al. (J. Am. Chem. Soc. 1963, 85, 14, 2149-2154), the following various epitope peptides (comprising mimotopes) of human αSyn and an AJ peptide were synthesized. Specifically, a protected peptide resin was synthesized by a Fmoc method using a fully automated solid-phase synthesizer according to methods disclosed in documents "Solid Phase Peptide Synthesis, Pierce (1984)" and "Fmoc solid synthesis: a practical approach, Oxford University Press (2000)," *"*Jikken Kagaku Koza (Encyclopedia 16 of Experimental Chemistry, 5th Ed.), Synthesis IV of Organic Compounds, edited by The Chemical Society of Japan, 2005," and the like. Trifluoroacetic acid (TFA) and a scavenger (a mixture of thioaniol, ethanedithiol, phenol, triisopropylsilane, water, and the like) were added to the obtained protected peptide resin, and deprotection was caused while cleaving from the resin to obtain a crude peptide. This crude peptide was purified by gradient elution with a 0.1% TFA-H2O/CH3CN system using a reverse-phase HPLC column (ODS). Fractions comprising a desired product were collected and freeze-dried to obtain a desired peptide. The amino acid sequence of the synthesized peptide was confirmed using an amino acid sequencer G1000A (Hewlett Packard), PPSQ-23A (Shimadzu Corporation), or ProciscLC (ABI), and the N-terminal of the obtained peptide was acetylated.
p54 (96-104aa of SEQ ID NO: 14): KKDQLGKNE (SEQ ID NO: 1)
p150 (123-131aa of SEQ ID NO: 14): EAYEMPSEE (SEQ ID NO: 2)
p283 (123-132aa of SEQ ID NO: 14, E131D): EAYEMPSEDG (SEQ ID NO: 3)
p142 (96-104aa of SEQ ID NO: 14, K102A): KKDQLGANE (SEQ ID NO: 4)
p167 (96-105aa of SEQ ID NO: 14): KKDQLGKNEE (SEQ ID NO: 5)
p227 (96-104aa of SEQ ID NO: 14, K97R): KRDQLGKNE (SEQ ID NO: 6)
p233 (96-104aa of SEQ ID NO: 14, E104D): KKDQLGKND (SEQ ID NO: 7)
p272 (96-104aa of SEQ ID NO: 14, D98N, E104D): KKNQLGKND (SEQ ID NO: 8)
p273 (96-104aa of SEQ ID NO: 14, D98T, E104D): KKTQLGKND (SEQ ID NO: 9)
p276 (123-132aa of SEQ ID NO: 14, E123D): DAYEMPSEEG (SEQ ID NO: 10)
p277 (123-132aa of SEQ ID NO: 14, Y125F): EAFEMPSEEG (SEQ ID NO: 11)
DMPVDPDN (115-122aa of SEQ ID NO: 14): DMPVDPDN (SEQ ID NO: 12)
AJ peptide: ELKLIFLHRLKRLRKRLKRK (SEQ ID NO: 13)

Subsequently, various conjugates shown in Table 1 below were produced. Regarding AJ-human αSyn peptide conjugates, the AJ peptide (SEQ ID NO: 13) and the various epitope peptides of human αSyn synthesized above were linked via ε-aminocaproic acid (ε-Acp) as a linker after adding a protective group, and condensation and deprotection were performed to produce various AJ-human αSyn peptide conjugates shown in Table 1. Furthermore, in the case of a conjugate to which KLH (Sigma-Aldrich) was linked, by covalently bonding cysteine as a linker and lysine residues present on the surface of KLH protein, linkage to a human αSyn peptide or an AJ-human αSyn peptide conjugate was performed, thereby producing a KLH-human αSyn peptide conjugate or a KLH-modified AJ-human αSyn peptide conjugate.

**[Table 1]**

| Conjugate | Amino acid sequence |
|---|---|
| AJ-p54 | ELKLIFLHRLKRLRKRLKRKXKKDQLGKNE |
| AJ-p150 | ELKLIFLHRLKRLRKRLKRKXEAYEMPSEE |
| AJ-p283 | ELKLIFLHRLKRLRKRLKRKXEAYEMPSEDG |
| KLH-AJ-p54 | KLH-C- ELKLIFLHRLKRLRKRLKRKXKKDQLGKNE |
| AJ-p54-KLH | ELKLIFLHRLKRLRKRLKRKXKKDQLGKNE-C- KLH |
| KLH-p54 | KLH-C-KKDQLGKNE |
| p54-KLH | KKDQLGKNE-C-KLH |
| AJ-p142 | ELKLIFLHRLKRLRKRLKRKXKKDQLGANE |
| AJ-p167 | ELKLIFLHRLKRLRKRLKRKXKKDQLGKNEE |
| AJ-p227 | ELKLIFLHRLKRLRKRLKRKXKRDQLGKNE |
| AJ-p233 | ELKLIFLHRLKRLRKRLKRKXKKDQLGKND |
| AJ-p272 | ELKLIFLHRLKRLRKRLKRKXKKNQLGKND |
| AJ-p273 | ELKLIFLHRLKRLRKRLKRKXKKTQLGKND |
| AJ-p276 | ELKLIFLBRLKRLRKRLKRKXDAYEMPSEEG |
| AJ-p277 | ELKLIFLHRLKRLRKRLKRKXEAFEMPSEEG |
| KLH-DMPVDPDN | KLH-C-DMPVDPDN |
| AJ-DMPVDPDN | ELKLIFLHRLKRLRKRLKRKXDMPVDPDN |

| | |
|---|---|
| X: ε-Acp, C: cysteine, KLH: keyhole limpet hemocyanin | |

### Example 2: Examination (1) of antibody-inducing ability of AJ peptide-human αSyn peptide conjugate with respect to human αSyn and aggregated human αSyn

Using the various AJ peptide-human αSyn peptide conjugates produced in Example 1, an antibody-inducing ability with respect to human αSyn in mice was evaluated. The various AJ-human αSyn peptide conjugates (250 µg/mouse) or KLH-DMPVDPDN (25 µg/mouse) in physiological saline were intramuscularly administered to balb/c mice (females, 6 weeks old, CLEA Japan, Inc.) twice at a 2-week interval for immunization using aluminum hydroxide (Alum) (InvivoGen, 50 µg/mouse) as an adjuvant. As a negative control, physiological saline (100 µL/mouse) comprising the same amount of aluminum hydroxide was administered twice. Two weeks after the second administration, serum was recovered to evaluate the antibody titer with respect to human αSyn by ELISA.

ELISA was performed by the following method. 96-well plates were coated with human αSyn (Sigma) at 0.2 µg/well. After washing with PBST (Phosphate Buffered Saline comprising 0.05% Tween) once, blocking was performed with 3% skim milk (FUJIFILM Wako Pure Chemical Corporation). After washing with PBST three times, diluted serum was added to cause a reaction at room temperature for 1 hour. Furthermore, after washing with PBST three times, Goat anti-mouse IgG-HRP (abcam) was added to cause a reaction at room temperature for 1 hour. After washing with PBST three times, SureBlue TMB Microwell Peroxidase Substrate (KPL) was added to cause a reaction at room temperature for 10 minutes while light shielding. An equal volume of a TMB Stop Solution (KPL) was added to stop the reaction, and the absorbance at 450 nm was measured. ELISA with respect to aggregated human αSyn was performed by coating with the aggregated human αSyn (Stress Marq) at 15 ng/well instead of human αSyn in the above-mentioned method.

Table 2 shows the results. Table 2 confirmed that the administration of the various AJ-human αSyn peptide conjugate induced the antibody to human αSyn in the mouse serum. In addition, regarding KLH-DMPVDPDN disclosed in Patent Literature 2, the induction of the antibody to human αSyn was also confirmed. Furthermore, regarding the aggregated human αSyn, although a particularly strong antibody-inducing ability was confirmed for AJ-p167, AJ-p227, AJ-p272, and AJ-p283, the antibody-inducing ability of KLH-DMPVDPDN was very weak.

**[Table 2]**

| Table 2. Antibody-inducing ability of human αSyn peptide conjugate with respect to human αSyn | | |
|---|---|---|
| Antigen | Antibody titer with respect to human αSyn (N = 4 - 6, mean ± standard deviation) | Antibody titer with respect to aggregated human αSyn (N = 4 - 6, mean ± standard deviation) |
| Physiological saline | 8 ± 0 | 16 ± 0 |
| AJ-p54 | 4096 ± 2896 | 768 ± 862 |
| AJ-p142 | 4693 ± 6431 | 576 ± 732 |
| AJ-p150 | 2176 ± 1471 | 576 ± 322 |
| AJ-p167 | 2389 ± 1399 | 1664 ± 1409 |
| AJ-p227 | 4267 ± 3200 | 3072 ± 2748 |
| AJ-p233 | 1323 ± 1505 | 555 ± 377 |
| AJ-p272 | 4096 ± 2896 | 1536 ± 1724 |
| AJ-p273 | 3328 ± 3278 | 704 ± 896 |
| AJ-p276 | 1792 ± 512 | 896 ± 256 |
| AJ-p277 | 1920 ± 1585 | 576 ± 322 |
| AJ-p283 | 2304 ± 1289 | 1152 ± 644 |
| KLH-DMPVDPDN | 5461 ± 2365 | 208 ± 205 |

### Example 3: Examination of antibody-inducing ability of AJ-human αSyn peptide conjugate and KLH-modified AJ-human αSyn peptide conjugate

Using the KLH-human αSyn peptide conjugates (KLH-p54 and p54-KLH) produced in Example 1, and KLH-AJ-human αSyn peptide conjugates (AJ-p54-KLH, KLH-AJ-p54), the antibody-inducing ability of these conjugates in the mice with respect to human αSyn was evaluated in the same manner as in Example 2. AJ-p54 was used as a comparison.

Table 3 shows the results. From Table 3, it was confirmed that the antibody to human αSyn was induced to the same extent even when using any vaccine of the AJ-human αSyn peptide conjugate (AJ-p54) and the KLH-modified AJ-human αSyn peptide conjugate (AJ-p54-KLH, KLH-AJ-p54). Regarding the KLH-modified AJ-human αSyn peptide conjugate, no difference in antibody titers was observed whether KLH was linked to the N-terminal or linked to the C-terminal. Furthermore, even when only KLH was linked and no AJ peptide was linked (KLH-p54, p54-KLH), the antibody to human αSyn was induced, and there was no difference in antibody titers whether KLH was linked to the N-terminal or linked to the C-terminal.

**[Table 3]**

| Table 3. Antibody-inducing ability of AJ-human αSyn peptide conjugate and KLH-modified AJ-human αSyn peptide conjugate with respect to human αSyn | |
|---|---|
| Name | Antibody titer (N = 4 - 6, mean ± standard deviation) |
| Physiological saline | 8 ± 0 |
| AJ-p54 | 1434 ± 1510 |
| AJ-p54-KLH | 1621 ± 681 |
| KLH-AJ-p54 | 1707 ± 1240 |
| KLH-p54 | 4096 ± 2243 |
| p54-KLH | 3072 ± 2748 |

### Example 4: Examination (2) of antibody-inducing ability of AJ-human αSyn peptide conjugate with respect to human αSyn and aggregated human αSyn

The antibody-inducing ability of the AJ-human αSyn peptide conjugates (AJ-p54, AJ-p54-KLH, and AJ-DMPVDPDN) produced in Example 1 with respect to human αSyn and aggregated human αSyn in mice was evaluated. Physiological saline or the AJ-human αSyn peptide conjugate (250 µg/mouse) was intramuscularly administered to balb/c mice (females, 6 weeks old, CLEA Japan, Inc.) twice at a 2-week interval using Alum (InvivoGen, 50 µg/mouse) as an adjuvant. Furthermore, KLH-DMPVDPDN (25 µg/mouse) was administered using Alum (InvivoGen, 50 µg/mouse) as an adjuvant. Two weeks after the second administration, serum was recovered to evaluate the antibody titer by ELISA. ELISA was performed by the method described in Example 2.

Table 4 shows the results. From Table 4, it was confirmed that AJ-p54 and AJ-p54-KLH induced the antibodies to human αSyn and aggregated human αSyn in the mice. However, although KLH-DMPVDPDN induced the antibody to human αSyn, it did not induce the antibody to aggregated human αSyn. In addition, AJ-DMPVDPDN exhibited a very weak antibody-inducing effect with respect to the antibodies to both human αSyn and aggregated human αSyn.

**[Table 4]**

| Table 4. Antibody-inducing ability of AJ-human αSyn peptide conjugate with respect to human αSyn and aggregated human αSyn | | |
|---|---|---|
| Name | Antibody titer with respect to human αSyn (mean ± standard deviation) | Antibody titer with respect to aggregated human αSyn (mean ± standard deviation) |
| Physiological saline | 8 ± 0 | 16 ± 0 |
| AJ-p54 | 1434 ± 1510 | 768 ± 296 |
| AJ-p54-KLH | 1621 ± 681 | 640 ± 314 |
| KLH-DMPVDPDN | 1792 ± 1536 | 36 ± 20 |
| AJ-DMPVDPDN | 168 ± 122 | 358 ± 140 |

### Example 5: Examination of human αSyn-neutralizing effect of antibody induced in mice by AJ-human αSyn peptide conjugate

The aggregated human αSyn-neutralizing effect of the antibody induced in the mice by the AJ-human αSyn peptide conjugate (AJ-p54) produced in Example 1 was evaluated. Physiological saline or the AJ-human αSyn peptide conjugate (250 µg/mouse) was intramuscularly administered to C57BL/6 mice (males, 6 weeks old, CLEA Japan, Inc.) four times at a 2-week interval using CFA (DIFCO) as an adjuvant. Furthermore, KLH-DMPVDPDN (25 µg/mouse) was administered using Alum (InvivoGen, 50 µg/mouse) as an adjuvant. Two weeks after the fourth administration, serum was recovered. After recovering the antibodies comprised in the serum by Protein G HP Spin Trap (Cytiva), the human αSyn- and aggregated human αSyn-neutralizing effect was evaluated by a method described below.

As the human αSyn- and aggregated human αSyn-neutralizing effect, the influence on phosphorylation of human αSyn in SH-SY5Y cells (ATCC) was evaluated. The SH-SY5Y cells were seeded in a 96-well plate at 2 × 10⁴ cells/well and cultured under the conditions of 37°C and 5% CO₂. On the next day, human αSyn (FUJIFILM Wako Pure Chemical Corporation) or aggregated human αSyn (Stress Marq) at different concentrations (5, 10, 20, 25 µg/mL) was added and cultured for another 3 days. After fixing the cells with a 4% paraformaldehyde/phosphate buffer solution (FUJIFILM Wako Pure Chemical Corporation), phosphorylated human αSyn was stained with a fluorescence-labeled anti-phosphorylated αSyn antibody (Cell Signaling). The amount of phosphorylated human αSyn was evaluated by image analysis using ArrayScan (Thermo Fisher Scientific Inc.).

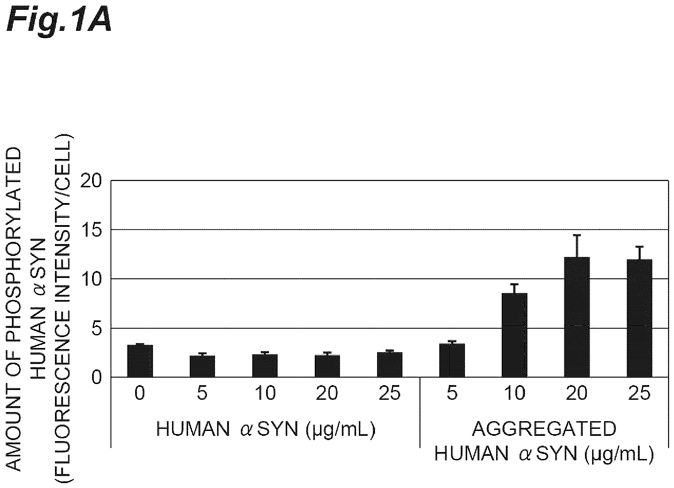

FIG. 1A shows the results (N = 3, mean ± standard error). The phosphorylation of human αSyn was observed in the SH-SY5Y cells to which aggregated human αSyn was added, but the phosphorylation of human αSyn was not recognized in the SH-SY5Y cells to which human αSyn was added.

Therefore, the inhibition of the phosphorylation of human αSyn in the SH-SY5Y cells to which aggregated human αSyn was added was examined. The aggregated human αSyn was mixed with the antibody (100 µg/mL) induced by the vaccine obtained above or a positive control antibody, and shaken at room temperature for 1 hour. As the positive control antibody, an Anti-α-synuclein filament antibody (abeam, clone MJFR-14-6-4-2, 10 µg/mL) was used (referred to as MJFR). The mixture was added to SH-SY5Y cells and cultured for 1 day under the conditions of 37°C and 5% CO₂, and the amount of phosphorylated human αSyn was measured in the same manner as above.

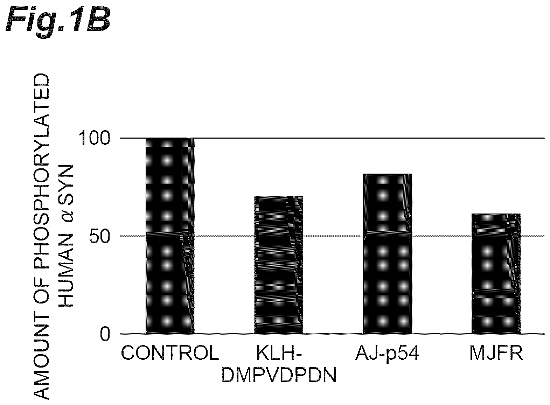

FIG. 1B shows the results (N = 3, mean ± standard error). In FIG. 1B, the amount of phosphorylated human αSyn was shown as 100 when an isotype control was added. The phosphorylation of human αSyn was inhibited in the SH-SY5Y cells to which the antibodies induced by AJ-p54 and KLH-DMPVDPDN were added. Because monomers are partially present in the aggregated human αSyn used in the present examination, it is thought that there is an equilibrium relationship with the aggregated type. Therefore, it is probable that the phosphorylation is inhibited even by the antibody derived from KLH-DMPVDPDN, which has a strong antibody-inducing effect with respect to monomers. From the above results, it was clarified that the antibodies induced by these vaccines have an aggregated human αSyn-neutralizing effect.

### Example 6: Human αSyn propagation inhibitory effect in human αSyn-overexpressing mice

The influence of the antibodies induced by AJ-human αSyn peptide conjugates (AJ-p54, AJ-p150) on the amount of human αSyn in the brain of Parkinson's disease model mice was examined. Physiological saline or the AJ-human αSyn peptide conjugate (250 µg/mouse) was intramuscularly administered to human αSyn-overexpressing mice (16 weeks old) four times at a 2-week interval using CFA (DIFCO) as an adjuvant. Furthermore, KLH-DMPVDPDN (25 µg/mouse) was administered using Alum (InvivoGen, 50 µg/mouse) as an adjuvant. The human αSyn-overexpressing mice produced according to the method disclosed in Non-Patent Literature 4 were used. Two weeks after the fourth administration, the cortex and the olfactory bulb were recovered. The amount of human αSyn in a soluble fraction in the cortex and the olfactory bulb was evaluated by a dot blot.

Sample preparation and the dot blot were performed as follows. Each of the cortex and the olfactory bulb of the mice was put in a ceramic beads kit tube CK14 (M&S Instruments Inc.), and ice-cold 1% Triton X-100, 10 mM Tris-HCl (pH 7.5), 150 mM NaCl, 5 mM EDTA, and Protease and Phosphatase Inhibitor Cocktail (Thermo Fisher Scientific Inc.) were added. Homogenization was performed with Micro Smash MS-100R (TOMY DIGITAL BIOLOGY CO., LTD.) for 30 seconds under the condition of 5,500 rpm. Furthermore, sonication was performed with a BIORUPTOR (BM Equipment Co., Ltd.). After leaving to stand for 30 minutes under ice-cooling, centrifugation was performed at 18,900 × g and 4°C for 20 minutes. The supernatant was recovered and used as a soluble fraction. A protein concentration was quantitatively determined using a BCA Protein Assay Kit (Thermo Fisher Scientific Inc.).

A membrane was set in a Dot Blot apparatus (Bio-Dot, Bio-Rad Laboratories, Inc.), and 0.5 µg/well of the soluble fractions of the cortex and the olfactory bulb was added. After leaving to stand at room temperature for 1 hour, suction filtration was performed. Blocking One (Nacalai Tesque, Inc.) was added, and after leaving to stand at room temperature for 1 hour, blocking was performed by suction filtration. After washing with PBS, an Anti-α-synuclein filament antibody (Abeam plc., clone MJFR-14-6-4-2) was added, and after leaving to stand at room temperature for 2 hours, suction filtration was performed. After washing with PBST, a Goat Anti-rabbit IgG and an HRP-linked Antibody (Cell Signaling Technology, Inc.) were added, and after leaving to stand at room temperature for 1 hour, suction filtration was performed. After washing with PBST, chemiluminescence was detected using Chemi-Lumi One Super (Nacalai Tesque, Inc.).

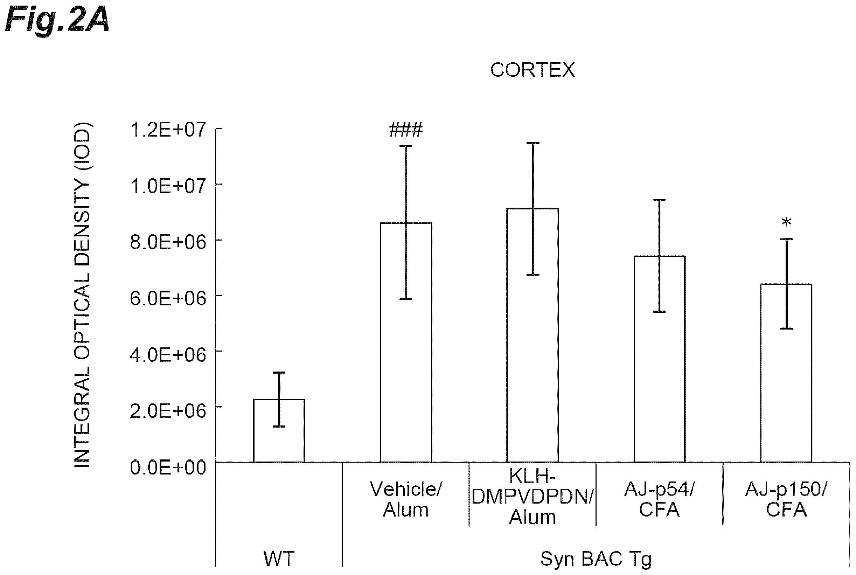

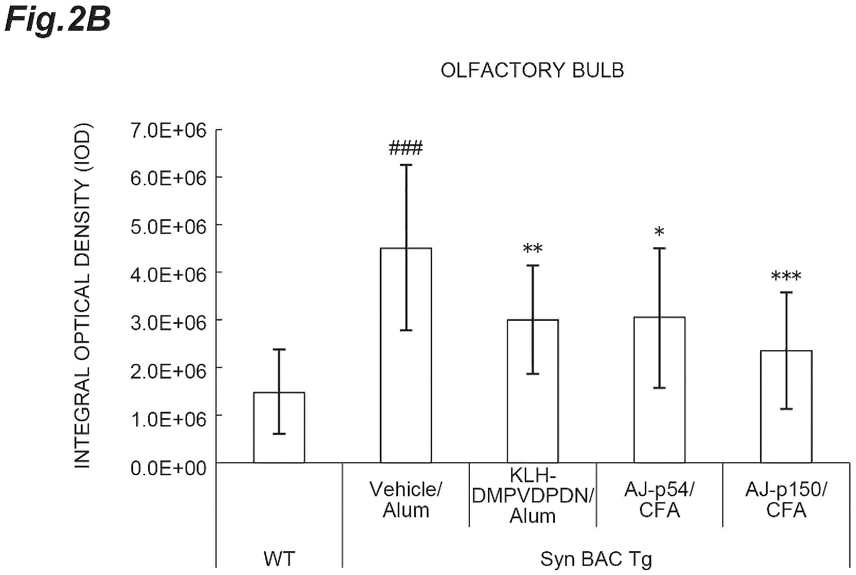

The results are shown in FIG. 2A (cortex) and FIG. 2B (olfactory bulb) (in FIGS. 2A and 2B, ###: p < 0.005 with respect to WT in a Welch's t-test; *: p < 0.05 with respect to Vehicle, **: p < 0.01 with respect to Vehicle, and ***: p < 0.005 with respect to Vehicle in a Dunnett's test; and Vehicle: no antigen). In the cortex, the administration of AJ-p150 significantly decreased the amount of human αSyn. Furthermore, in the olfactory bulb, the administration of AJ-p54, AJ-p150, and KLH-DMPVDPDN significantly decreased the amount of human αSyn. From the above results, it was clarified that the AJ-human αSyn peptide conjugate exhibits a human αSyn propagation inhibitory effect in the human αSyn-overexpressing mice.

In addition, the soluble fractions of the cortex and the olfactory bulb were analyzed by a western blot. Specifically, 10 µg of the soluble fractions of the cortex and the olfactory bulb was subjected to SDS-PAGE using a 15% to 20% polyacrylamide gel (FUJIFILM Wako Pure Chemical Corporation) and Tris/Tricine/SDS buffer (Bio-Rad Laboratories, Inc.). The gel was placed on the membrane of Trans-Blot Turbo Mini 0.2 µm PVDF Transfer Packs (Bio-Rad Laboratories, Inc.) to transfer proteins to the membrane using a blotting device Trans-Blot Turbo (Bio-Rad Laboratories, Inc.). The membrane was moved to a container, to which a 4% paraformaldehyde phosphate buffer solution (FUJIFILM Wako Pure Chemical Corporation) was added, and shaken at room temperature for 30 minutes. After washing the membrane with PBS three times by shaking it for 5 minutes, Blocking One (Nacalai Tesque, Inc.) was added, and blocking was performed by shaking at room temperature for 30 minutes. A solution of a primary antibody alpha Synuclein Monoclonal Antibody (clone: Syn211, Thermo Fisher Scientific Inc.) diluted 10,000-fold with Blocking One diluted 10-fold with PBST was added to the membrane and shaken overnight at 4°C. After washing the membrane with PBST three times by shaking for 5 minutes, a solution of a secondary antibody Goat anti-Mouse IgG (H + L) Cross-Adsorbed Secondary Antibody, HRP (Thermo Fisher Scientific Inc.) diluted 20,000-fold using Blocking One diluted 10-fold with PBST was added and shaken at room temperature for 1 hour. After washing the membrane with PBST three times by shaking for 5 minutes, chemiluminescence was detected using Chemi-Lumi One Super (Nacalai Tesque, Inc.).

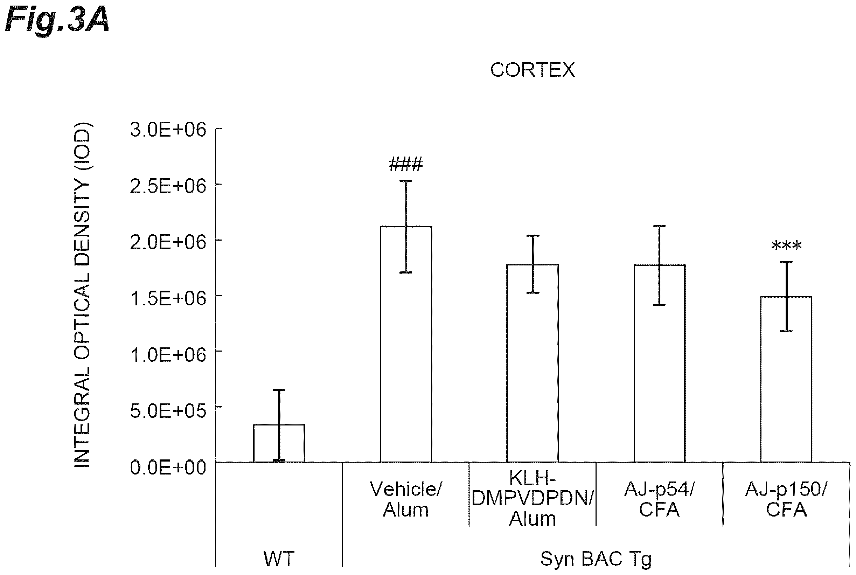

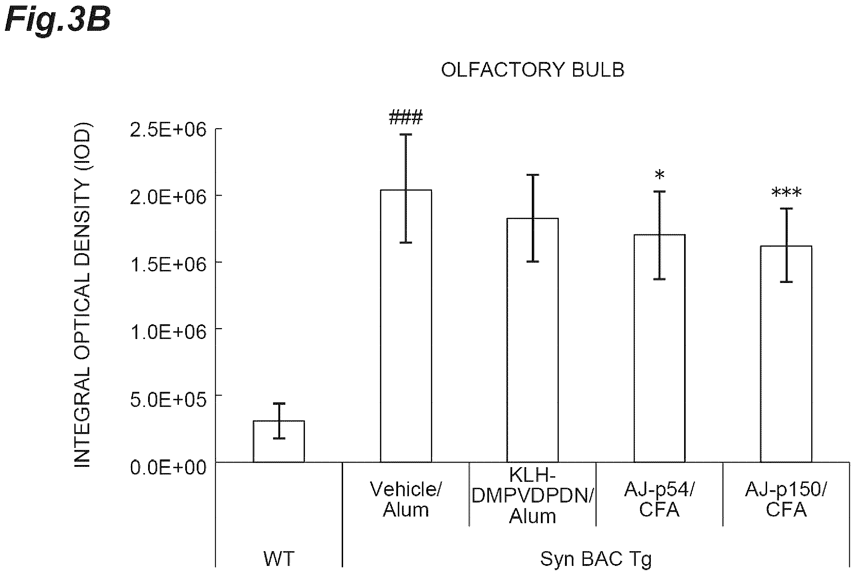

The results are shown in FIG. 3A (cortex) and FIG. 3B (olfactory bulb) (in FIGS. 3A and 3B, ###: p < 0.005 with respect to WT in a Welch's t-test; *: p < 0.05 with respect to Vehicle, and ***: p < 0.005 with respect to Vehicle in a Dunnett's test; and Vehicle: no antigen). In the cortex, the administration of AJ-p150 significantly decreased the amount of human αSyn. Furthermore, in the olfactory bulb, the administration of AJ-p54 and AJ-p150 significantly decreased the amount of human αSyn, but a significantly decrease in the amount of human αSyn was not shown by the administration of KLH-DMPVDPDN.

### Example 7: Examination of antibody-inducing ability with respect to human αSyn and aggregated human αSyn in cynomolgus monkeys

The antibody-inducing ability of the AJ-human αSyn peptide conjugate with respect to human αSyn and aggregated human αSyn in cynomolgus monkeys was evaluated. The AJ-human αSyn peptide conjugates (AJ-p54, AJ-p150, AJ-p283, AJ-p142, and AJ-p273) (2,000 µg/monkey) and KLH-DMPVDPDN (75 µg/monkey) were intramuscularly administered to cynomolgus monkeys (males, 3 to 7 years old, SNBL, Ltd.) thee times at a 2-week interval using Alum (InvivoGen, 200 µg/monkey) as an adjuvant. Blood was collected before administration and 4, 6, 8, and 10 weeks after the first administration to recover the serum. ELISA was performed by the following method.

96-well plates were coated with human αSyn (Sigma-Aldrich) or aggregated human αSyn (StressMarq Biosciences Inc.) at 0.2 µg/well or 15 ng/well. After washing with PBST once, blocking was performed with 3% skim milk (FUJIFILM Wako Pure Chemical Corporation). After washing with PBST three times, diluted serum was added to cause a reaction at room temperature for 1 hour. Furthermore, after washing with PBST three times, Goat anti-Monkey IgG-HRP (Bio-Rad Laboratories, Inc.) was added to cause a reaction at room temperature for 1 hour. After washing with PBST three times, SureBlue^{™} TMB Microwell Peroxidase Substrate (KPL) was added to cause a reaction at room temperature for 10 minutes while light shielding. An equal volume of a TMB Stop Solution (KPL) was added to stop the reaction, and the absorbance at 450 nm was measured.

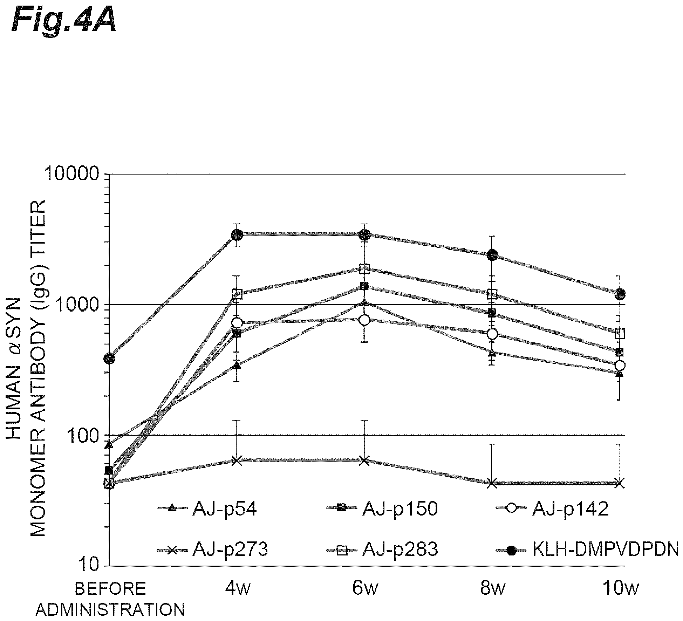

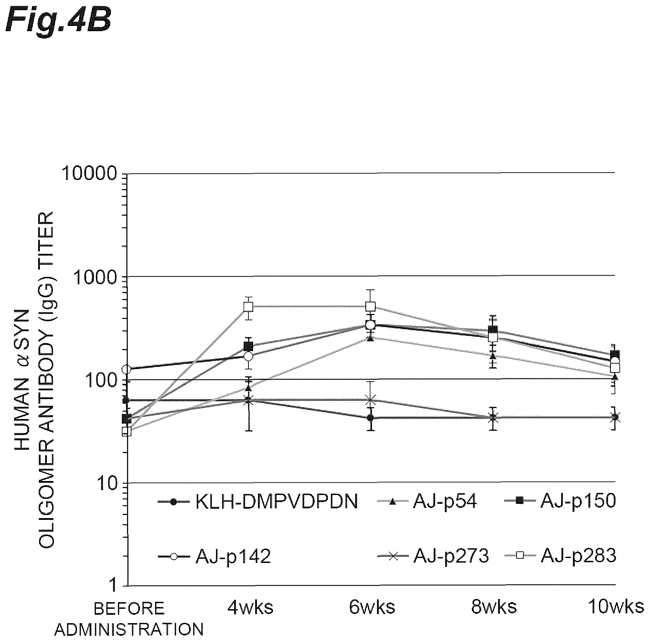

FIG. 4A shows the antibody titers with respect to human αSyn, and FIG. 4B shows the antibody titers with respect to aggregated human αSyn (N = 3, mean ± standard error). All of the vaccines induced the antibodies to human αSyn. The antibody to aggregated human αSyn was not induced by KLH-DMPVDPDN and AJ-p273, but was induced by the vaccines other than these.

### Example 8: Examination of human αSyn-neutralizing effect of antibody induced in cynomolgus monkeys

The aggregated human αSyn-neutralizing effect of the antibody induced in the cynomolgus monkeys by the AJ-human αSyn peptide conjugate was evaluated. The AJ-human αSyn peptide conjugates (AJ-p54 and AJ-p150, 2,000 µg/monkey) and KLH-DMPVDPDN (75 µg/monkey) were intramuscularly administered to cynomolgus monkeys (males, 3 to 7 years old, SNBL, Ltd.) thee times at a 2-week interval using Alum (InvivoGen, 200 µg/monkey) as an adjuvant. Two weeks after the third administration, the serum was recovered. After recovering the antibodies comprised in the serum by Protein G HP Spin Trap (Cytiva), the aggregated human αSyn-neutralizing effect was evaluated by the method described in Example 5. As the positive control antibody, an Anti-α-synuclein filament antibody (Abeam plc., clone MJFR-14-6-4-2, 10 µg/mL) was used (referred to as MJFR).

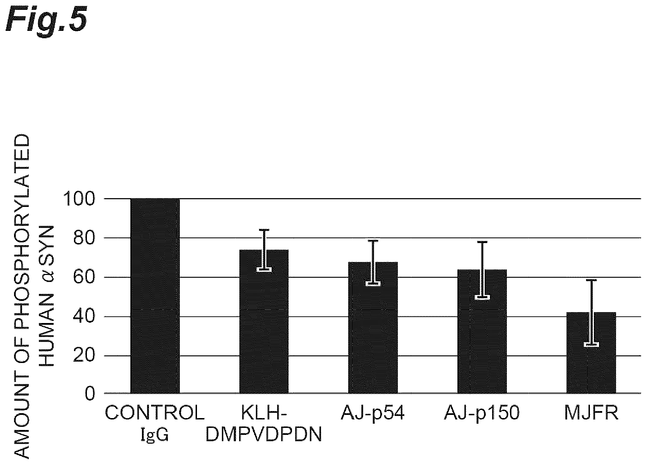

FIG. 5 shows the results. In FIG. 5, the amount of phosphorylated αSyn was shown as 100 when isotype control IgG (100 µg/mL) was added. The antibodies induced by any of vaccines inhibited the phosphorylation of human αSyn in the SH-SY5Y cells. From the above description, it was clarified that the antibodies induced by these vaccines have an aggregated human αSyn-neutralizing effect.

### Example 9: Examination (3) of antibody-inducing ability of AJ-human αSyn peptide conjugate with respect to human αSyn and aggregated human αSyn

The following peptides were synthesized by the method described in Example 1, and then various human αSyn peptide conjugates shown in Table 5 below were produced by the method described in Example 1.
p150 (123-131aa of SEQ ID NO: 14): EAYEMPSEE (SEQ ID NO: 2)
p242 (123-132aa of SEQ ID NO: 14, E123A): AAYEMPSEEG (SEQ ID NO: 18)
p244 (123-132aa of SEQ ID NO: 14, Y125A): EAAEMPSEEG (SEQ ID NO: 19)
p347 (123-132aa of SEQ ID NO: 14, E123H, Y125F): HAFEMPSEEG (SEQ ID NO: 20)
p361 (123-132aa of SEQ ID NO: 14, E123D, Y125F): DAFEMPSEEG (SEQ ID NO: 21)
p54 (96-104aa of SEQ ID NO: 14): KKDQLGKNE (SEQ ID NO: 1)
p229 (96-104aa of SEQ ID NO: 14, D98T): KKTQLGKNE (SEQ ID NO: 22)
p237 (96-104aa of SEQ ID NO: 14, D98N): KKNQLGKNE (SEQ ID NO: 23)
p298 (96-104aa of SEQ ID NO: 14, Q99E, N103D): KKDELGKDE (SEQ ID NO: 24)

**[Table 5]**

| Conjugate | Amino acid sequence |
|---|---|
| AJ-p150 | ELKLIFLHRLKRLRKRLKRKXEAYEMPSEE |
| AJ-p242 | ELKLIFLHRLKRLRKRLKRKXAAYEMPSEEG |
| AJ-p244 | ELKLIFLHRLKRLRKRLKRKXEAAEMPSEEG |
| AJ-p347 | ELKLIFLHRLKRLRKRLKRKXHAFEMPSEEG |
| AJ-p361 | ELKLIFLHRLKRLRKRLKRKXDAFEMPSEEG |
| AJ-p54 | ELKLIFLHRLKRLRKRLKRKXKKDQLGKNE |
| AJ-p229 | ELKLIFLHRLKRLRKRLKRKXKKTQLGKNE |
| AJ-p237 | ELKLIFLHRLKRLRKRLKRKXKKNQLGKNE |
| AJ-p298 | ELKLIFLHRLKRLRKRLKRKXKKDELGKDE |

| | |
|---|---|
| X: ε-Acp | |

The antibody-inducing ability of the produced various human αSyn peptide conjugates with respect to human αSyn and aggregated human αSyn in the mice was evaluated by the method described in Example 2 using the antibody titers with respect to human αSyn and aggregated human αSyn of the mouse immune serum. Table 6 shows the results. In Table 6, the antibody titer of AJ-p242, AJ-p244, AJ-p347, or AJ-p361 is shown with the antibody titer of AJ-p150 as a control being 1 (logarithm). In addition, the antibody titers of AJ-p229, AJ-p237, and AJ-p298 are shown with the antibody titer of AJ-p54 as a control being 1 (logarithm).

**[Table 6]**

| Table 6. Antibody-inducing ability of human αSyn peptide conjugate with respect to human αSyn and aggregated human αSyn | | |
|---|---|---|
| Name | Antibody titer with respect to human αSyn | Antibody titer with respect to aggregated human αSyn |
| AJ-p 150 | 1 | 1 |
| AJ-p242 | 1.06 | 1.12 |
| AJ-p244 | 1.06 | 1.10 |
| AJ-p347 | 0.97 | 0.97 |
| AJ-p361 | 0.92 | 0.95 |
| AJ-p54 | 1 | 1 |
| AJ-p229 | 0.92 | 1.02 |
| AJ-p237 | 0.91 | 0.95 |
| AJ-p298 | 0.82 | 0.93 |

| | | |
|---|---|---|
| N=4 | | |

From Table 6, it was clarified that the produced various AJ-human αSyn peptide conjugates (AJ-p242, AJ-p244, AJ-p347, AJ-p361, AJ-p229, AJ-p237, and AJ-p298) showed the same level of an antibody titer as the antibody titer of the AJ-human αSyn peptide conjugate as a control.

### Example 10: Examination of antibody-inducing ability of AJ-human αSyn peptide conjugate with modified linker with respect to human αSyn and aggregated human αSyn

A linker portion was modified in AJ-p54 and AJ-p150 to produce various AJ-human αSyn peptide conjugates with a modified linker shown in Table 7 below.

**[Table 7]**

| Table 7. AJ-human αSyn peptide conjugate with modified linker | |
|---|---|
| Name | Amino acid sequence |
| AJ-G-p54 | ELKLIFLHRLKRLRKRLKRK**G**KKDQLGKNE |
| AJ-εK-p54 | ELKLIFLHRLKRLRKRLKRK**εK**KKDQLGKNE |
| AJ-αK-p54 | ELKLIFLHRLKRLRKRLKRK**αK**KKDQLGKNE |
| AJ-εKKK-p54 | ELKLIFLHRLKRLRKRLKRK**εKKK**KKDQLGKNE |
| AJ-εKKKK-p54 | ELKLIFLBRLKRLRKRLKRK**εKKKK**KKKDQLGKNE |
| AJ-GGGGS-p54 | ELKLIFLHRLKRLRKRLKRK**GGGGS**KKDQLGKNE |
| AJ-EAAAK-p54 | ELKLIFLHRLKRLRKRLKRK**EAAAK**KKDQLGKNE |
| AJ-DDAKK-p54 | ELKLIFLHRLKRLRKRLKRK**DDAKK**KKDQLGKNE |
| AJ-PPDPDP-p54 | ELKLIFLHRLKRLRKRLKRK**PPDPDP**KKDQLGKNE |
| AJ-G-p150 | ELKLIFLHRLKRLRKRLKRK**G**EAYEMPSEE |
| AJ-εK-p150 | ELKLIFLHRLKRLRKRLKRK**εK**EAYEMPSEE |
| AJ-αK-p150 | ELKLIFLBRLKRLRKRLKRK**αK**EAYEMPSEE |
| AJ-εKKK-p150 | ELKLIFLHRLKRLRKRLKRK**εKKK**EAYEMPSEE |
| AJ-εKKKK-p150 | ELKLIFLHRLKRLRKRLKRK**εKKKK**EAYEMPSEE |
| AJ-GGGGS-p150 | ELKLIFLHRLKRLRKRLKRK**GGGGS**EAYEMPSEE |
| AJ-EAAAK-p150 | ELKLIFLHRLKRLRKRLKRK**EAAAK**EAYEMPSEE |
| AJ-DDAKK-p150 | ELKLIFLBRLKRLRKRLKRK**DDAKK**EAYEMPSEE |
| AJ-PPDPDP-p150 | ELKLIFLBRLKRLRKRLKRK**PPDPDP**EAYEMPSEE |

| | |
|---|---|
| εK: indicating that AJ peptide is bound to ε-position of lysine αK: indicating that AJ peptide is bound to α-position of lysine | |

The antibody-inducing ability of the AJ-human αSyn peptide conjugates with a modified linker with respect to human αSyn and aggregated human αSyn in the mice was evaluated using the antibody titers with respect to human αSyn and aggregated human αSyn of the mouse immune serum. Table 8 shows the results. In Table 8, the antibody titers of the AJ-human αSyn peptide conjugates with various modified linkers comprising p54 are shown with the antibody titer of AJ-p54 as a control being 1 (logarithm). In addition, the antibody titers of the AJ-human αSyn peptide conjugates with various modified linkers comprising p150 are shown with the antibody titer of AJ-p150 as a control being 1 (logarithm).

**[Table 8]**

| Table 8. Antibody-inducing ability of AJ-human αSyn peptide conjugate with modified linker with respect to human αSyn and aggregated human αSyn | | |
|---|---|---|
| Name | Antibody titer with respect to human αSyn | Antibody titer with respect to aggregated human αSyn |
| AJ-p54 | 1 | 1 |
| AJ-G-p54 | 0.89 | 0.96 |
| AJ-εK-p54 | 0.70 | 0.81 |
| AJ-αK-p54 | 0.99 | 0.92 |
| AJ-εKKK-p54 | 0.74 | 0.88 |
| AJ-εKKKK-p54 | 0.77 | 0.90 |
| AJ-GGGGS-p54 | 1.05 | 1.02 |
| AJ-EAAAK-p54 | 0.96 | 0.96 |
| AJ-DDAKK-p54 | 1.03 | 0.98 |
| AJ-PPDPDP-p54 | 0.89 | 0.88 |
| AJ-p 150 | 1 | 1 |
| AJ-G-p150 | 0.96 | 0.95 |
| AJ-εK-p150 | 0.82 | 0.86 |
| AJ-αK-p150 | 0.95 | 1.05 |
| AJ-εKKK-p150 | 0.79 | 0.90 |
| AJ-εKKKK-p150 | 0.97 | 1.00 |
| AJ-GGGGS-p150 | 1.19 | 1.25 |
| AJ-EAAAK-p150 | 1.14 | 1.16 |
| AJ-DDAKK-p150 | 1.14 | 1.30 |
| AJ-PPDPDP-p150 | 1.33 | 1.37 |

From Table 8, it was confirmed that the AJ-human αSyn peptide conjugates with various modified linkers showed the same level of an antibody titer as that of AJ-p54 or AJ-p150 which are conjugates with ε-Acp as a linker. From the above results, it was clarified that these AJ-human αSyn peptide conjugates induced antibodies even when the linker portion was modified.

## Claims

1. A polypeptide consisting of any of the following amino acid sequences:
(a) an amino acid sequence set forth in SEQ ID NO: 1;
(b) an amino acid sequence set forth in SEQ ID NO: 2; and
(c) an amino acid sequence in which one, two, or three amino acids are substituted, deleted, added, and/or inserted in the amino acid sequence set forth in SEQ ID NO: 1 or SEQ ID NO: 2.

2. The polypeptide according to claim 1, wherein the amino acid sequence is the amino acid sequence set forth in SEQ ID NO: 1.

3. The polypeptide according to claim 1, wherein the amino acid sequence is the amino acid sequence set forth in SEQ ID NO: 2.

4. The polypeptide according to claim 1, wherein the amino acid sequence is an amino acid sequence set forth in any one of SEQ ID NOs: 4 to 9 and SEQ ID NOs: 22 to 24.

5. The polypeptide according to claim 1, wherein the amino acid sequence is an amino acid sequence set forth in any one of SEQ ID NO: 3, SEQ ID NO: 10, SEQ ID NO: 11, and SEQ ID NOs: 18 to 21.

6. A conjugate comprising the polypeptide according to any one of claims 1 to 5 and a carrier protein.

7. The conjugate according to claim 6, wherein the carrier protein is keyhole limpet hemocyanin (KLH).

8. The conjugate according to claim 6, wherein the carrier protein is a peptide (AJ peptide) consisting of an amino acid sequence identical or substantially identical to an amino acid sequence set forth in SEQ ID NO: 13.

9. The conjugate according to claim 8, wherein the AJ peptide and the polypeptide are linked via ε-aminocaproic acid and/or a peptide linker.

10. The conjugate according to claim 9, wherein the peptide linker is a peptide linker consisting of one to six L-amino acids or a repeating sequence thereof.

11. The conjugate according to any one of claims 8 to 10, wherein an N-terminal amino acid of the conjugate is acetylated.

12. The conjugate according to any one of claims 8 to 11, wherein a C-terminal amino acid of the conjugate is amidated.

13. A pharmaceutical composition comprising the polypeptide according to any one of claims 1 to 5 or the conjugate according to any one of claims 6 to 12.

14. The pharmaceutical composition according to claim 13, further comprising an adjuvant.

15. The pharmaceutical composition according to claim 13 or 14, which is a vaccine for preventing, ameliorating, or treating a neurodegenerative disease.

16. The pharmaceutical composition according to claim 15, wherein the neurodegenerative disease is a disease associated with α-synuclein accumulation.

17. The pharmaceutical composition according to claim 16, wherein the disease associated with the α-synuclein accumulation is Parkinson's disease.
